# EUROPEAN PATENT APPLICATION

(11) **EP 4 844 063 A1**
(43) Date of publication of application: **25.11.2026**
(21) Application number: 25741628.9
(22) Date of filing: 17.01.2025
(51) Int. Cl.: C12N 15/113, A61K 48/00, A61P 25/00, A61P 29/00

(54) **NUCLEIC ACID FOR INHIBITING SODIUM VOLTAGE-GATED CHANNEL ALPHA SUBUNIT 10 (SCN10A) GENE EXPRESSION, AND USE THEREOF**

(30) Priority: 19.01.2024 CN 202410084366
(71) Applicant: YUN HO BIO CO., LTD, Shanghai 201206 (CN)
(72) Inventor: ZHU, Yun, Shanghai 201206 (CN); WU, Yun, Shanghai 201206 (CN); LIU, Yang, Shanghai 201206 (CN); MAO, Song, Shanghai 201206 (CN); DONG, Xianglei, Shanghai 201206 (CN)
(74) Representative: Simmons & Simmons LLP (Munich)
(86) International application number: PCT/CN2025/073030
(87) International publication number: WO 2025/153066

(57) **Abstract**

Disclosed are an antisense oligonucleotide or RNAi agent for inhibiting SCN10A gene expression in cells, and a pharmaceutical composition thereof. Also disclosed is the therapeutic use of the antisense oligonucleotide and the RNAi agent.

## Description

### Technical Filed

The present invention relates to nucleic acids that inhibit the expression of sodium voltage-gated channel alpha subunit 10 (SCN10A) gene in cells, including antisense oligonucleotides (ASO) and RNAi agents (such as siRNA), and pharmaceutical compositions comprising the same. The invention also relates to therapeutic uses of the nucleic acids.

### Background

An ion channel is an integral membrane protein containing a water-filled pore that regulates the membrane voltage potential by modulating ion flux across the cell membrane. Studies have shown that alterations in ion channels constitute the molecular basis of peripheral sensitization, central sensitization, and disinhibition following inflammatory or neuropathic injury, and represent an important molecular mechanism of pain generation. There are nine subtypes of human sodium channels, namely Nav1.1-Nav1.9, among which Nav1.8 is encoded by the SCN10A (Sodium Voltage-Gated Channel Alpha Subunit 10) gene and is predominantly expressed in the trigeminal ganglion (TRG) and dorsal root ganglion (DRG). Nav1.8 is a critical ion channel involved in chronic neuropathic and chronic inflammatory pain, atrial fibrillation, and Brugada syndrome, and serves as a highly selective therapeutic target for pain management.

Therefore, it is anticipated that targeting the SCN10A gene with siRNA or ASO drugs, thereby achieving efficient and specific inhibition of the target gene's mRNA, pre-mRNA, and protein, may provide beneficial effects in the treatment (including prophylactic treatment) of acute and chronic pain, such as traumatic and postoperative pain, nociceptive pain (e.g., pain associated with osteoporosis and rheumatoid arthritis), neuropathic pain (central types, such as post-stroke pain, spinal cord injury, migraine, HIV-associated neuropathic pain, etc.; peripheral types, such as postherpetic neuralgia, diabetic neuropathy), visceral pain (e.g., pain arising from pancreatitis, inflammatory bowel syndrome, etc.), and mixed pain (e.g., low back pain, cancer pain, fibromyalgia, etc.), and related conditions.

Currently, no small nucleic acid-based drugs targeting this gene have been approved for marketing, and there remains a need to develop such drugs with improved efficacy, prolonged durability, specific targeting, and/or safety.

### Summary

The present invention is directed to targeting the SCN10A gene via nucleic acid therapeutics (e.g., siRNA or ASO), enabling specific and potent suppression of target genes and proteins in target organs, thereby treating acute and chronic pain.

One aspect of the present invention provides an antisense oligonucleotide, wherein the antisense oligonucleotide comprises an oligonucleotide consisting of 12 to 30 contiguous nucleotides, and wherein the oligonucleotide comprises at least 8, at least 9, at least 10, at least 11, at least 12, at least 13, at least 14, at least 15, at least 16, at least 17, at least 18, at least 19, or at least 20 contiguous nucleotides of any one of the nucleotide sequences set forth in SEQ ID NOs: 3 to 20.

In some embodiments, when measured over the entire nucleotide sequence of the antisense oligonucleotide, the antisense oligonucleotide has a nucleotide sequence that is at least 80%, at least 85%, at least 90%, at least 95% or 100% complementary to the nucleotide sequence set forth in SEQ ID NO. 1 and/or SEQ ID NO. 2.

In some embodiments, the antisense oligonucleotide comprises at least one modification selected from a modified sugar moiety, a modified nucleobase moiety, and a modified internucleoside linkage. Preferably, the antisense oligonucleotide comprises one or more of the following modifications: (i) at least one bicyclic sugar moiety, wherein the bicyclic sugar moiety preferably has a 4'-2' bridge, and the 4'-2' bridge is preferably cEt or LNA; (ii) at least one non-bicyclic modified sugar moiety, preferably selected from 2'-deoxy, 2'-F, 2'-MOE, and 2'-OMe sugar moieties; (iii) a modified nucleoside, preferably comprising 5-methylcytosine (m5C); and (iv) at least one sugar surrogate, preferably selected from POM, PNA, THP, and F-HNA.

In some embodiments, the antisense oligonucleotide comprises a gapmer. In some embodiments, the gapmer comprises: (a) a 5' region consisting of 1 to 6 linked 5' region nucleotides; (b) a central region consisting of 6 to 10 linked central region nucleotides; and (c) a 3' region consisting of 1 to 6 linked 3' region nucleotides; wherein each of the nucleotides in the 5' region and the 3' region comprises a sugar moiety selected from 2'-MOE, LNA, and cEt modified sugar moieties, and at least 6 of the nucleotides in the central region comprise a 2'-deoxy sugar moiety.

In some embodiments, the antisense oligonucleotide has a sugar motif selected from the group consisting of the following (5' to 3'): eekddddddddddkke, ekkddddddddddkke, kkkdyddddddddkkk, kkkddydddddddkkk, kkkdddyddddddkkk, kkkddddddddddkkk, and eeeeeeddddddddddeeeee; wherein e represents a 2'-MOE sugar moiety, k represents a cEt sugar moiety, d represents a 2'-deoxy sugar moiety, and y represents a 2'-OMe sugar moiety.

In some embodiments, the antisense oligonucleotide comprises at least one modified internucleoside linkage; preferably, each internucleoside linkage of the antisense oligonucleotide is a modified internucleoside linkage; preferably, the modified internucleoside linkage is selected from phosphorothioate (PS) and phosphoramidate (PN) (e.g., methanesulfonyl phosphoramidate (MsPA)), preferably PS.

In some embodiments, the antisense oligonucleotide comprises an oligonucleotide consisting of 12-30, 14-22, 14-20, 14-18, 14-20, 15-17, 15-25, or 16-20 linked nucleotides, preferably, the oligonucleotide consists of 20 contiguous nucleotides; preferably, the antisense oligonucleotide comprises, or consists of, a nucleotide sequence as set forth in any one of SEQ ID NOs: 21 to 38, or a nucleotide sequence having 1 to 3 nucleotide differences therefrom.

In some embodiments, the antisense oligonucleotide comprises a ligand; preferably, wherein the ligand comprises a ligand targeting hepatocytes, preferably, the ligand comprises a galactose moiety, a galactosamine moiety, or an N-acetylgalactosamine moiety, more preferably, the ligand is a trivalent or tetravalent N-acetylgalactosamine moiety, and even more preferably, the hepatocyte-targeting ligand is L96, NAG25, or NAG37; or the ligand comprises a ligand targeting non-hepatocyte cells, an integrin ligand, or a transferrin receptor 1 ligand, preferably the ligand is a lipophilic group. The lipophilic group is preferably selected from: lipids, vitamins, steroids, C₅-C₃₀ saturated or unsaturated fatty acids, C₅-C₃₀ alkyl groups, and polypeptides comprising at least one positively charged amino acid residue; the lipophilic group is more preferably selected from cholesterol, C₁₆ saturated or unsaturated fatty acid, C₁₆ alkyl group, C₂₂ saturated or unsaturated fatty acid, or C₂₂ alkyl group.

Another aspect of the present invention provides an RNAi agent for inhibiting expression of sodium voltage-gated channel alpha subunit 10 (SCN10A) gene in a cell, comprising a sense strand and an antisense strand forming a duplex region, wherein the antisense strand comprises at least 15 contiguous nucleotides of a nucleotide sequence selected from SEQ ID NOs: 59 to 78 and nucleotide sequences having 1 to 3 nucleotide differences therefrom.

In some embodiments, the duplex region is 17 to 23 base pairs in length, preferably 18 to 21 base pairs, more preferably 19 base pairs.

In some embodiments, each of the sense strand and the antisense strand is 17 to 23 nucleotides in length, preferably 19 to 21 nucleotides.

In some embodiments, the RNAi agent comprises one or two blunt ends, preferably one blunt end.

In some embodiments, the RNAi agent comprises one or two overhangs, preferably one overhang, each overhang comprising 1 to 4 unpaired nucleotides, preferably 2 unpaired nucleotides.

In some embodiments, the overhang is located at 3'-end of the sense strand, 3'-end of the antisense strand, or simultaneously at 3'-end of the sense strand and 3'-end of the antisense strand; preferably, the overhang is located at 3'-end of the antisense strand, and more preferably, the RNAi agent has one blunt end.

In some embodiments, the sense strand comprises at least 15 contiguous nucleotides of any nucleotide sequence selected from SEQ ID NOs: 39 to 58 and nucleotide sequences having 1 to 3 nucleotide differences therefrom.

In some embodiments, the antisense strand has no more than 23 nucleotides and comprises a nucleotide sequence selected from SEQ ID NOs: 59 to 78; and the sense strand has no more than 21 nucleotides and comprises a nucleotide sequence from SEQ ID NOs: 39 to 58.

In some embodiments, in the RNAi agent:
the sense strand comprises or consists of a sequence set forth in SEQ ID NO:39 or a nucleotide sequence having 1, 2, or 3 nucleotide differences therefrom, and the antisense strand comprises or consists of a sequence set forth in SEQ ID NO:59 or a nucleotide sequence having 1, 2, or 3 nucleotide differences therefrom;
the sense strand comprises or consists of a sequence set forth in SEQ ID NO:40 or a nucleotide sequence having 1, 2, or 3 nucleotide differences therefrom, and the antisense strand comprises or consists of a sequence set forth in SEQ ID NO:60 or a nucleotide sequence having 1, 2, or 3 nucleotide differences therefrom;
the sense strand comprises or consists of a sequence set forth in SEQ ID NO:41 or a nucleotide sequence having 1, 2, or 3 nucleotide differences therefrom, and the antisense strand comprises or consists of a sequence set forth in SEQ ID NO:61 or a nucleotide sequence having 1, 2, or 3 nucleotide differences therefrom;
the sense strand comprises or consists of a sequence set forth in SEQ ID NO:42 or a nucleotide sequence having 1, 2, or 3 nucleotide differences therefrom, and the antisense strand comprises or consists of a sequence set forth in SEQ ID NO:62 or a nucleotide sequence having 1, 2, or 3 nucleotide differences therefrom;
the sense strand comprises or consists of a sequence set forth in SEQ ID NO:43 or a nucleotide sequence having 1, 2, or 3 nucleotide differences therefrom, and the antisense strand comprises or consists of a sequence set forth in SEQ ID NO:63 or a nucleotide sequence having 1, 2, or 3 nucleotide differences therefrom;
the sense strand comprises or consists of a sequence set forth in SEQ ID NO:44 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom;
the sense strand comprises or consists of a sequence set forth in SEQ ID NO:45 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom;
the sense strand comprises or consists of a sequence set forth in SEQ ID NO:46 or a nucleotide sequence having 1, 2, or 3 nucleotide differences therefrom, and the antisense strand comprises or consists of a sequence set forth in SEQ ID NO:66 or a nucleotide sequence having 1, 2, or 3 nucleotide differences therefrom;
the sense strand comprises or consists of a sequence set forth in SEQ ID NO:47 or a nucleotide sequence having 1, 2, or 3 nucleotide differences therefrom, and the antisense strand comprises or consists of a sequence set forth in SEQ ID NO:67 or a nucleotide sequence having 1, 2, or 3 nucleotide differences therefrom;
the sense strand comprises or consists of a sequence set forth in SEQ ID NO:48 or a nucleotide sequence having 1, 2, or 3 nucleotide differences therefrom, and the antisense strand comprises or consists of a sequence set forth in SEQ ID NO:68 or a nucleotide sequence having 1, 2, or 3 nucleotide differences therefrom;
the sense strand comprises or consists of a sequence set forth in SEQ ID NO:49 or a nucleotide sequence having 1, 2, or 3 nucleotide differences therefrom, and the antisense strand comprises or consists of a sequence set forth in SEQ ID NO:69 or a nucleotide sequence having 1, 2, or 3 nucleotide differences therefrom;
the sense strand comprises or consists of a sequence set forth in SEQ ID NO:50 or a nucleotide sequence having 1, 2, or 3 nucleotide differences therefrom, and the antisense strand comprises or consists of a sequence set forth in SEQ ID NO:70 or a nucleotide sequence having 1, 2, or 3 nucleotide differences therefrom;
the sense strand comprises or consists of a sequence set forth in SEQ ID NO:51 or a nucleotide sequence having 1, 2, or 3 nucleotide differences therefrom, and the antisense strand comprises or consists of a sequence set forth in SEQ ID NO:71 or a nucleotide sequence having 1, 2, or 3 nucleotide differences therefrom;
the sense strand comprises or consists of a sequence set forth in SEQ ID NO:52 or a nucleotide sequence having 1, 2, or 3 nucleotide differences therefrom, and the antisense strand comprises or consists of a sequence set forth in SEQ ID NO:72 or a nucleotide sequence having 1, 2, or 3 nucleotide differences therefrom;
the sense strand comprises or consists of a sequence set forth in SEQ ID NO:53 or a nucleotide sequence having 1, 2, or 3 nucleotide differences therefrom, and the antisense strand comprises or consists of a sequence set forth in SEQ ID NO:73 or a nucleotide sequence having 1, 2, or 3 nucleotide differences therefrom;
the sense strand comprises or consists of a sequence set forth in SEQ ID NO:54 or a nucleotide sequence having 1, 2, or 3 nucleotide differences therefrom, and the antisense strand comprises or consists of a sequence set forth in SEQ ID NO:74 or a nucleotide sequence having 1, 2, or 3 nucleotide differences therefrom;
the sense strand comprises or consists of a sequence set forth in SEQ ID NO:55 or a nucleotide sequence having 1, 2, or 3 nucleotide differences therefrom, and the antisense strand comprises or consists of a sequence set forth in SEQ ID NO:75 or a nucleotide sequence having 1, 2, or 3 nucleotide differences therefrom;
the sense strand comprises or consists of a sequence set forth in SEQ ID NO:56 or a nucleotide sequence having 1, 2, or 3 nucleotide differences therefrom, and the antisense strand comprises or consists of a sequence set forth in SEQ ID NO:76 or a nucleotide sequence having 1, 2, or 3 nucleotide differences therefrom;
the sense strand comprises or consists of a sequence set forth in SEQ ID NO:57 or a nucleotide sequence having 1, 2, or 3 nucleotide differences therefrom, and the antisense strand comprises or consists of a sequence set forth in SEQ ID NO:77 or a nucleotide sequence having 1, 2, or 3 nucleotide differences therefrom; or
the sense strand comprises or consists of a sequence set forth in SEQ ID NO:58 or a nucleotide sequence having 1, 2, or 3 nucleotide differences therefrom, and the antisense strand comprises or consists of a sequence set forth in SEQ ID NO:78 or a nucleotide sequence having 1, 2, or 3 nucleotide differences therefrom.

In some embodiments, the RNAi agent comprises duplex 51059, 51069, 51071, 51078, 51089, 51120, 51154, 51201, 51264, 51284, 51286, 51329, 51346, 51350, 51370, 51407, 51415, 51417, 51435, or 51441.

In some embodiments, the sense strand and/or the antisense strand of the RNAi agent comprises at least one modified nucleotide, the modified nucleotide independently selected from 2'-deoxy-thymidine (dT) nucleotide, 2'-O-methyl-modified nucleotide (2'-OMe), 2'-fluoro-modified nucleotide (2'-F), 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide (cEt), 2'-amino-modified nucleotide, 2'-O-allyl-modified nucleotide, 2'-C-alkyl-modified nucleotide, 2'-O-methoxyethyl-modified nucleotide (2'-MOE), abasic nucleotide, inverted abasic nucleotide, inverted nucleotide, morpholino nucleotide (MOP), phosphoramidate (PN), tetrahydropyran-modified nucleotide (THP), 1,5-anhydrohexitol-modified nucleotide (HNA), cyclohexenyl-modified nucleotide, nucleotide comprising a phosphorothioate group (PS), nucleotide comprising a methylphosphonate group, nucleotide comprising a 5'-phosphate, nucleotide comprising a 5'-phosphate mimic, cationic lipid-conjugated nucleotide, nucleotide comprising a 5'-vinylphosphonate (5'-VP), and combinations thereof; preferably selected from 2'-O-methyl-modified nucleotide, 2'-fluoro-modified nucleotide, nucleotide comprising a phosphorothioate internucleotide linkage, and combinations thereof; and/or preferably, each nucleotide of the sense strand and/or the antisense strand of the RNAi agent is modified.

In some embodiments, in the RNAi agent, in 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, in the RNAi agent, the antisense strand comprises at least one phosphorothioate internucleotide linkage; preferably, the phosphorothioate internucleotide linkage is present at one or more of the following locations: (i) between nucleotide positions 1 and 2 at 5'-end of the antisense strand; (ii) between nucleotide positions 2 and 3 at 5'-end of the antisense strand; (iii) between nucleotide positions 1 and 2 at 3'-end of the antisense strand; and (iv) between nucleotide positions 2 and 3 at 3'-end of the antisense strand.

In some embodiments, in the RNAi agent, in 5' to 3' direction, the nucleotides at positions 7 and 9 of the sense strand are 2'-fluoro-modified nucleotides, and one or two of the nucleotides at positions 5, 8, and 11 of the sense strand are 2'-fluoro-modified nucleotides, while the nucleotides at remaining positions of the sense strand are all 2'-methoxy-modified nucleotides.

In some embodiments, the sense strand has at least one phosphorothioate internucleoside linkage; preferably, the phosphorothioate internucleoside linkage is present at one or more of the following positions: (i) between the 1st nucleotide and the 2nd nucleotide at the 5' end of the sense strand; (ii) between the 2nd nucleotide and the 3rd nucleotide at the 5' end of the sense strand; (iii) between the 1st nucleotide and the 2nd nucleotide at the 3' end of the sense strand; and (iv) between the 2nd nucleotide and the 3rd nucleotide at the 3' end of the sense strand.

In some embodiments, the modification is selected from one of STC, ESC, Advanced ESC, ESC+, AD1-3, AD5 and GalXC.

In some embodiments, the modification is that the 5'-terminal nucleotide of the antisense strand contains a phosphate or phosphate analog modification, preferably 5'-VP.

In some embodiments, the RNAi agent comprises a ligand targeting hepatocytes, preferably, the ligand comprises a galactose moiety, a galactosamine moiety, or an N-acetylgalactosamine moiety, more preferably, the ligand is a trivalent or tetravalent N-acetylgalactosamine moiety, and even more preferably, the hepatocyte-targeting ligand is L96, NAG25, or NAG37. Alternatively, the RNAi agent comprises a ligand targeting non-hepatocyte cells, preferably a lipophilic group, an integrin ligand, or a transferrin receptor 1 ligand. The lipophilic group is preferably selected from: lipids, vitamins, steroids, C₅-C₃₀ saturated or unsaturated fatty acids, C₅-C₃₀ alkyl groups, and polypeptides comprising at least one positively charged amino acid residue; the lipophilic group is more preferably selected from cholesterol, C₁₆ saturated or unsaturated fatty acid, C₁₆ alkyl group, C₂₂ saturated or unsaturated fatty acid, or C₂₂ alkyl group.

A further aspect of the present invention provides a pharmaceutical composition comprising any ASO or RNAi agent of the present invention and a pharmaceutically acceptable carrier; preferably, the pharmaceutical composition is formulated for intravenous or subcutaneous injection.

Another aspect of the present invention provides the use of any RNAi agent of the present invention in the manufacture of a medicament for:
(i) reducing expression level of SCN10A in a cell;
(ii) preventing or treating a disease mediated by aberrant expression levels of SCN10A; or
(iii) prevention or treatment of a disease or condition selected from acute and chronic pain, such as traumatic and postoperative pain, nociceptive pain (e.g., pain caused by osteoporosis and rheumatoid arthritis), neuropathic pain (central types, such as post-stroke pain, spinal cord injury, migraine, HIV-associated neuropathic pain, etc.; peripheral types, such as postherpetic neuralgia and diabetic neuropathy), visceral pain (e.g., pain arising from pancreatitis, inflammatory bowel syndrome, etc.), and mixed pain (e.g., low back pain, cancer pain, fibromyalgia, etc.).

Accordingly, the present invention also provides a method of reducing SCN10A expression level in a cell, the method comprising administering to a subject in need thereof a therapeutically effective amount of any one of the antisense oligonucleotides or any one of the RNAi agents of the present invention. The present invention also provides a method for preventing or treating a disease mediated by aberrant SCN10A expression level (e.g., caused by elevated levels thereof), the method comprising administering to a subject in need thereof a therapeutically effective amount of any one of the antisense oligonucleotides or any one of the RNAi agents of the present invention. The present invention also provides a method for preventing or treating a disease selected from acute and chronic pain, such as traumatic and postoperative pain, nociceptive pain (e.g., pain caused by osteoporosis and rheumatoid arthritis), neuropathic pain (central types, such as post-stroke pain, spinal cord injury, migraine, HIV-associated neuropathic pain, etc.; peripheral types, such as postherpetic neuralgia and diabetic neuropathy), visceral pain (e.g., pain arising from pancreatitis, inflammatory bowel syndrome, etc.), and mixed pain (e.g., low back pain, cancer pain, fibromyalgia, etc.), the method comprising administering to a subject in need thereof a therapeutically effective amount of any one of the antisense oligonucleotides or any one of the RNAi agents of the present invention. The present invention also provides use of any one of the antisense oligonucleotides or any one of the RNAi agents of the present invention in the manufacture of a medicament for the prevention or treatment of the above-mentioned diseases. The present invention also provides any one of the antisense oligonucleotides or any one of the RNAi agents of the present invention for use in the prevention or treatment of the above-mentioned diseases.

Other aspects of the invention will be apparent from the detailed description of the specification that follows.

### Detailed Description of the Invention

### Definition

Sodium voltage-gated channel alpha subunit 10 (SCN10A), as used herein, refers to the sodium voltage-gated channel alpha subunit 10 protein or its encoding gene, also known as FEPS2, Nav1.8, PN3, or SNS, with Gene ID: 6336. The NCBI accession number for the full-length human SCN10A mRNA transcript is NM_006514.4. The NCBI accession number for the cynomolgus monkey SCN10A mRNA transcript is XM_005546684.3.

As used herein, "oligonucleotide" refers to a nucleotide sequence that forms a chain of nucleotides via internucleoside linkages, wherein each nucleoside and internucleoside linkage may be modified or unmodified. Unless otherwise indicated, the oligonucleotide consists of 12 to 30 linked nucleotides. As used herein, "antisense oligonucleotide" or "ASO" refers to a single-stranded oligonucleotide capable of specifically binding to a target RNA (e.g., mRNA) sequence and modulating the expression of a protein (e.g., SCN10A). As described herein, the "antisense oligonucleotide" may comprise a non-oligonucleotide moiety, such as a ligand.

"Internucleoside linkage" refers to a covalent linkage between adjacent nucleotides in an oligonucleotide. As used herein, "modified internucleoside linkage" refers to any internucleoside linkage other than a phosphodiester internucleoside linkage. "Phosphorothioate internucleoside linkage" is a modified internucleoside linkage in which one non-bridging oxygen atom of the phosphodiester internucleoside linkage is replaced by a sulfur atom.

"Deoxy region" refers to a region of 5 to 12 contiguous nucleotides, wherein at least 70% of the nucleosides are 2'-β-D-deoxynucleosides. In certain embodiments, each nucleoside is selected from 2'-β-D-deoxynucleosides, bicyclic nucleosides, and 2'-substituted nucleosides. In certain embodiments, the deoxy region supports RNase H activity. In certain embodiments, the deoxy region is the gap or internal region of a gapmer.

"Gapmer" refers to an antisense oligonucleotide comprising an internal region flanked by external regions each having one or more nucleosides, wherein the internal region comprises a plurality of nucleosides that support RNase H cleavage, and wherein the nucleosides constituting the internal region are chemically distinct from the nucleoside(s) constituting the external regions. The internal region may be referred to as the "gap," and the external regions may be referred to as the "wings." The internal region is a deoxy region. The position of the internal region or gap refers to the order of the nucleosides in the internal region and is counted from the 5' end of the internal region. Unless otherwise indicated, "gapmer" refers to a sugar motif. In certain embodiments, each nucleoside of the gap is a 2'-β-D-deoxynucleoside. In certain embodiments, the gap comprises one 2'-substituted nucleoside at position 1, 2, 3, 4, or 5 of the gap, and the remaining nucleosides of the gap are 2'-β-D-deoxynucleosides. As used herein, the term "MOE gapmer" indicates a gapmer having a gap comprising 2'-β-D-deoxynucleosides and wings comprising 2'-MOE nucleosides. As used herein, the term "mixed wing gapmer" refers to a gapmer having wings that comprise modified nucleosides containing at least two different sugar modifications. Unless otherwise indicated, a gapmer may comprise one or more modified internucleoside linkages and/or modified nucleobases, and such modifications do not necessarily follow the sugar-modified gapmer pattern.

As used herein, the term "RNAi agent" refers to a reagent comprising an RNA molecule that, when introduced into a cell, is capable of down-regulating the expression of a target gene (in this context, the SCN10A gene) through an RNA interference mechanism. The terms "RNAi agent of the present invention," "RNAi agent described herein," or similar expressions encompass both the modified RNAi agents of the present invention, irrespective of sequence and target gene, and the RNAi agents of the present invention with specific sequences for interfering with the SCN10A gene. RNA interference refers to a process whereby a nucleic acid molecule induces cleavage and degradation of a target RNA molecule (e.g., an mRNA molecule) in a sequence-specific manner, such as through the RNA-induced silencing complex (RISC) pathway. RNAi agents herein include siRNA, shRNA, and DNA/RNA hybrid molecules, also sometimes collectively referred to as double-stranded RNA (dsRNA), which comprise two antiparallel continuous nucleotide strands sufficiently complementary to hybridize and form a double-stranded region. "Hybridization" refers to the pairing of complementary polynucleotides, typically through hydrogen bonding between complementary bases in the two polynucleotides (e.g., Watson-Crick hydrogen bonding, Wobble hydrogen bonding, Hoogsteen hydrogen bonding, or reverse Hoogsteen hydrogen bonding). The "double-stranded region" refers to an area within two complementary or substantially complementary polynucleotides that form base pairs through hybridization, thereby creating a duplex between the two polynucleotide strands.

The term "antisense strand" refers to the strand in a dsRNA that contains a region substantially complementary to a target sequence. The term "sense strand" or "passenger strand" refers to the strand in a dsRNA that contains a region substantially complementary to the antisense strand region as defined herein. The term "region substantially complementary" denotes a region that is either perfectly complementary or imperfectly complementary. When the complementary region is imperfectly complementary to the target sequence, mismatches may occur in internal or terminal regions of the molecule. Typically, the most tolerated mismatches are located in terminal regions, for example, within 5, 4, 3, or 2 nucleotides at the 5' and/or 3' termini of the dsRNA.

"siRNA" refers to a nucleic acid that forms a double-stranded RNA capable of reducing or inhibiting the expression of a target gene when present in the same cell as the target gene. An siRNA is typically approximately 15 to approximately 30 base pairs in length, most typically approximately 19 to 25 base pairs in length, for example, 19, 20, 21, 22, 23, 24, or 25 nucleotide pairs in length.

"shRNA" refers to short hairpin RNA, which comprises two short inverted repeats and an intervening stem-loop structure connecting them. The stem-loop may comprise at least one unpaired nucleotide, for example, at least 2, at least 3, at least 4, at least 5, at least 6, at least 7, at least 8, at least 9, at least 10, at least 20, at least 23, or more unpaired nucleotides. The stem-loop may be 10 or fewer nucleotides in length. The stem-loop may comprise 8 or fewer unpaired nucleotides. The stem-loop may comprise 4 to 10 unpaired nucleotides. The stem-loop may be 4 to 8 nucleotides in length.

The two substantially complementary strands of a dsRNA need not be, but may be, covalently linked. The maximum number of base pairs is the number of nucleotides in the shortest strand of the dsRNA minus any overhangs present in the duplex. In addition to the duplex structure, a dsRNA may comprise one or more nucleotide overhangs. An overhanging nucleotide refers to one or more unpaired nucleotides extending beyond the double-stranded region at the terminus of a strand. Nucleotide overhangs typically occur when the 3' end of one strand extends beyond the 5' end of the other strand, or when the 5' end of one strand extends beyond the 3' end of the other strand. For example, at least one strand comprises a 3' overhang of at least 1 nucleotide, e.g., 1 to 4 nucleotide overhangs. In another example, at least one strand comprises a 5' overhang of at least 1 nucleotide, e.g., 1 to 4 nucleotide overhangs. In other embodiments, both the 3' terminus and the 5' terminus of one strand of the dsRNA comprise an overhang of at least 1 nucleotide.

As used herein, the terms "blunt end" or "blunt terminus" with respect to dsRNA refer to the absence of unpaired nucleotides or nucleotide analogs at a given terminus of the dsRNA, i.e., no nucleotide overhang. One end or both ends of the dsRNA may be blunt. Where both ends of the dsRNA are blunt, the dsRNA is referred to as blunt-ended. It is to be expressly understood that a "blunt-ended" dsRNA is one having blunt termini at both ends, meaning no nucleotide overhang exists at either end of the molecule. In most cases, such molecules are double-stranded throughout their entire length. As used herein, the term "nucleotide overhang" refers to at least one unpaired nucleotide projecting from the duplex structure of the dsRNA. For example, a nucleotide overhang exists when the 3' terminus of one strand of the dsRNA extends beyond the 5' terminus of the other strand, or vice versa. The overhang may comprise or consist of nucleotides/nucleosides analogs, including deoxyribonucleotides/deoxyribonucleosides. Overhangs may be present on the sense strand, antisense strand, or any combination thereof. Furthermore, the overhanging nucleotides may reside at the 5' terminus, 3' terminus, or both termini of the antisense strand or sense strand of the dsRNA.

The dsRNA molecule may comprise chemical modifications to ribonucleotides, including modifications to the ribose, base, or backbone components of ribonucleic acid, as described herein or known in the art. Any such modifications, when used in double-stranded ribonucleic acid molecules (e.g., siRNA, shRNA, etc.), are encompassed by the term "dsRNA" for purposes of this disclosure. A "modified" nucleotide refers to a nucleotide that independently has a modified sugar moiety, a modified internucleoside linkage, and/or a modified nucleobase. Accordingly, the term "modified nucleotide" includes substitutions, additions, or removals of functional groups or atoms to the internucleoside linkage, sugar moiety, or nucleobase.

The term "ligand" refers to a cell or tissue targeting agent that binds to a specified cell type (e.g., hepatocytes), such as a lectin, glycoprotein, lipid, or protein (e.g., an antibody). Exemplary targeting agents include thyrotropin, melanotropin, lectins, glycoproteins, surfactant protein A, mucin carbohydrates, multivalent lactose, multivalent galactose, N-acetylgalactosamine (GalNAc), multivalent (e.g., bivalent or trivalent) GalNAc, N-acetylglucosamine, multivalent mannose, multivalent fucose, glycosylated polyamino acids, multivalent galactose, transferrin, bisphosphonates, polyglutamate, polyaspartate, cholesterol, steroids, bile acids, folate, vitamin B12, biotin, RGD peptides, and RGD peptide mimetics. In preferred embodiments, the ligand is a carbohydrate, such as a monosaccharide, disaccharide, trisaccharide, tetrasaccharide, or polysaccharide. For example, the ligand may be a derivative comprising GalNAc. In preferred embodiments, the ligand is a derivative comprising one or more N-acetylgalactosamine moieties attached via a bivalent or trivalent branched linker.

The term "therapeutically effective amount" refers to an amount of an RNAi agent or composition of the present invention that is effective to produce a desired therapeutic effect in at least a subpopulation of cells in an animal at a reasonable benefit/risk ratio applicable to any medical treatment.

The term "pharmaceutically acceptable" refers to compounds, materials, compositions, and/or dosage forms that are, within the scope of sound medical judgment, suitable for contact with tissues of humans and animals without excessive toxicity, irritation, allergic reactions, or other problems or complications and commensurate with a reasonable benefit/risk ratio.

The term "pharmaceutically acceptable carrier" refers to a pharmaceutically acceptable material, composition, or vehicle that participates in carrying or transporting an ASO or RNAi agent from one organ or part of the body to another organ or part of the body, such as a liquid or solid filler, diluent, excipient, manufacturing aid, or solvent encapsulating material. Each carrier must be "acceptable" in the sense of being compatible with other ingredients of the composition and innocuous to the patient.

The term "treatment" encompasses prevention, therapy, and cure. The patient receiving such treatment is typically any animal in need, including primates (particularly humans) and other mammals such as horses, cattle, swine, sheep, poultry, and companion animals.

### Antisense Oligonucleotides for Inhibiting SCN10A Gene Expression

A first aspect of the present invention provides an antisense oligonucleotide, comprising an oligonucleotide consisting of 12 to 30 contiguous nucleosides, said oligonucleotide comprising at least 8, at least 9, at least 10, at least 11, at least 12, at least 13, at least 14, at least 15, at least 16, at least 17, at least 18, at least 19, or at least 20 contiguous nucleosides of any one of SEQ ID NOs: 3 to 20.

In some embodiments, the oligonucleotide consists of 12-30, 14-22, 14-20, 14-18, 14-20, 15-17, 15-25 or 16-20 linked nucleotides and has a nucleotide sequence comprising at least 8, at least 9, at least 10, at least 11, at least 12, at least 13, at least 14, at least 15, at least 16, at least 17, at least 18, at least 19, or at least 20 contiguous nucleotides of the nucleotide sequence of any one of SEQ ID NOs: 3 to 20, and wherein when measured over the entire nucleotide sequence of the antisense oligonucleotide, the antisense oligonucleotide has a nucleotide sequence that is at least 80%, at least 85%, at least 90%, at least 95% or 100% (preferably 100%) complementary to the nucleotide sequence of the messenger RNA (mRNA) of human and/or cynomolgus monkey SCN10A (NCBI accession numbers NM_006514.4, SEQ ID NO. 1 and XM_005546684.3, SEQ ID NO. 2, respectively).

In some embodiments, the provided antisense oligonucleotides do not comprise modified nucleotides, such as modified sugar moieties, modified nucleobase moieties, or modified internucleoside linkages. In such embodiments, for example, the antisense oligonucleotide provided by the present invention may comprise the nucleotide sequence set forth in any one of SEQ ID NOs: 3, 4, 5, 6, 7, 8, 9, 10, 11, 12, 13, 14, 15, 16, 17, 18, 19, 20, or a nucleotide sequence having 1, 2, or 3 nucleotide differences therefrom.

In some embodiments, the provided antisense oligonucleotides comprise modified nucleotides, such as modified sugar moieties and/or modified nucleobase moieties and/or modified internucleoside linkages. In preferred embodiments, the provided antisense oligonucleotide comprises modified nucleotides, including modified sugar moieties, modified nucleobase moieties, and modified internucleoside linkages.

In some embodiments, the antisense oligonucleotide comprises an oligonucleotide consisting of 12-30, 14-22, 14-20, 14-18, 14-20, 15-17, 15-25, or 16-20 linked nucleosides, said oligonucleotide comprising at least 8, at least 9, at least 10, at least 11, at least 12, at least 13, at least 14, at least 15, at least 16, at least 17, at least 18, at least 19, or at least 20 contiguous nucleosides of the nucleotide sequence of any one of SEQ ID NOs: 3 to 20, wherein the antisense oligonucleotide comprises at least one modification selected from the group consisting of a modified sugar moiety, a modified nucleobase moiety, and a modified internucleoside linkage.

In some embodiments, every nucleoside in the oligonucleotide comprised in the antisense oligonucleotide is modified, said modification comprising a modified sugar moiety and a modified internucleoside linkage.

In some embodiments, every nucleoside in the oligonucleotide comprised in the antisense oligonucleotide is modified, said modification comprising a modified sugar moiety, a modified nucleobase moiety, and a modified internucleoside linkage.

In some embodiments, the modified sugar moiety may be at least one bicyclic sugar moiety, at least one non-bicyclic modified sugar moiety, and/or at least one sugar surrogate.

In some embodiments, the antisense oligonucleotide comprises at least one bicyclic sugar moiety. In some embodiments, a substituent bridges two atoms of a furanosyl ring to form a second ring, thereby generating a bicyclic sugar moiety. In certain such embodiments, the bicyclic sugar moiety comprises a bridge between the 4' and 2' furanosyl ring atoms. Examples of such 4' to 2' bridging sugar substituents include, but are not limited to: 4'-CH₂-2', 4'-(CH₂)₂-2', 4'-(CH₂)₃-2', 4'-CH₂-O-2' ("LNA"), 4'-CH₂-S-2', 4'-(CH₂)₂-O-2' ("ENA"), 4'-CH(CH₃)-O-2' (referred to as "constrained ethyl" or "cEt"), 4'-CH₂-O-CH₂-2', 4'-CH₂-N(R)-2', 4'-CH(CH₂OCH₃)-O-2' ("constrained MOE" or "cMOE") and analogs thereof (see, e.g., Seth et al., U.S. 7,399,845; Bhat et al., U.S. 7,569,686; Swayze et al., U.S. 7,741,457; and Swayze et al., U.S. 8,022,193), 4'-C(CH₃)(CH₃)-O-2' and analogs thereof (see, e.g., Seth et al., U.S. 8,278,283), 4'-CH₂-N(OCH₃)-2' and analogs thereof (see, e.g., Prakash et al., U.S. 8,278,425), 4'-CH₂-O-N(CH₃)-2' (see, e.g., Allerson et al., U.S. 7,696,345 and Allerson et al., U.S. 8,124,745), 4'-CH₂-C(H)(CH₃)-2' (see, e.g., Zhou et al., J. Org. Chem., 2009, 74, 118-134), 4'-CH₂-C(=CH₂)-2' and analogs thereof (see, e.g., Seth et al., U.S. 8,278,426), 4'-C(RₐR_{b})-N(R)-O-2', 4'-C(RₐR_{b})-O-N(R)-2', 4'-CH₂-O-N(R)-2', and 4'-CH₂-N(R)-O-2', wherein each R, Rₐ, and R_{b} is independently H, a protecting group, or C₁-C₁₂ alkyl (see, e.g., Imanishi et al., U.S. 7,427,672). In certain embodiments, bicyclic sugar moieties and nucleosides incorporating such bicyclic sugar moieties are further defined by isomeric configuration. For example, LNA nucleosides (as described herein) may be in the α-L configuration or in the β-D configuration. In preferred embodiments, the bicyclic sugar moiety has a 4'-2' bridge, which is preferably cEt or LNA.

In some embodiments, the antisense oligonucleotide comprises at least one non-bicyclic modified sugar moiety, and such non-bridging substituents may be located at any position of the furanosyl ring, including, but not limited to, substituents at the 2'-position, the 4'-position, and/or the 5'-position. In certain embodiments, one or more non-bridging substituents of the non-bicyclic modified sugar moiety are branched. Examples of 2'-substituents suitable for non-bicyclic modified sugar moieties include, but are not limited to: 2'-F, 2'-OCH₃ ("OMe" or "O-methyl"), and 2'-O(CH₂)₂OCH₃ ("MOE" or "O-methoxyethyl"). In certain embodiments, the 2'-substituent is selected from: halo, allyl, amino, azido, SH, CN, OCN, CF₃, OCF₃, O-C₁-C₁₀ alkoxy, substituted O-C₁-C₁₀ alkoxy, O-C₁-C₁₀ alkyl, substituted O-C₁-C₁₀ alkyl, S-alkyl, N(Rₘ)-alkyl, O-alkenyl, S-alkenyl, N(Rₘ)-alkenyl, O-alkynyl, S-alkynyl, N(Rₘ)-alkynyl, O-alkylene-O-alkyl, alkynyl, alkaryl, aralkyl, O-alkaryl, O-aralkyl, O(CH₂)₂SCH₃, O(CH₂)₂ON(Rₘ)(Rₙ), or OCH₂C(=O)-N(Rₘ)(Rₙ), wherein each Rₘ and Rₙ is independently H, an amino protecting group, or substituted or unsubstituted C₁-C₁₀ alkyl, as well as the 2'-substituents set forth in Cook et al., U.S. 6,531,584; Cook et al., U.S. 5,859,221; and Cook et al., U.S. 6,005,087. Certain embodiments of these 2'-substituents may be further substituted with one or more substituents independently selected from: hydroxyl, amino, alkoxy, carboxyl, benzyl, phenyl, nitro (NO₂), thiol, thioalkoxy, thioalkyl, halogen, alkyl, aryl, alkenyl, and alkynyl. Examples of 4'-substituents suitable for non-bicyclic modified sugar moieties include, but are not limited to, alkoxy (e.g., methoxy), alkyl, and those groups set forth in Manoharan et al., WO 2015/106128. Examples of 5'-substituents suitable for non-bicyclic modified sugar moieties include, but are not limited to: 5'-methyl (R or S), 5'-vinyl, and 5'-methoxy. In certain embodiments, the non-bicyclic modified sugar moiety comprises more than one non-bridging sugar substituent, such as a 2'-F-5'-methyl sugar moiety and modified sugar moieties and modified nucleosides as set forth in Migawa et al., WO 2008/101157 and Rajeev et al., US 2013/0203836.

In certain embodiments, the 2'-substituted non-bicyclic modified nucleoside comprises a sugar moiety comprising a non-bridging 2'-substituent selected from the group consisting of: F, NH₂, N₃, OCF₃, OCH₃, O(CH₂)₃NH₂, CH₂CH=CH₂, OCH₂CH=CH₂, OCH₂CH₂OCH₃, O(CH₂)₂SCH₃, O(CH₂)₂ON(Rₘ)(Rₙ), O(CH₂)₂O(CH₂)₂N(CH₃)₂, and N-substituted acetamide (OCH₂C(=O)-N(Rₘ)(Rₙ)), wherein each Rₘ and Rₙ is independently H, an amino protecting group, or substituted or unsubstituted C₁-C₁₀ alkyl.

In certain embodiments, the 2'-substituted non-bicyclic modified nucleoside comprises a sugar moiety comprising a non-bridging 2'-substituent selected from the group consisting of: F, OCF₃, OCH₃, OC H₂CH₂OCH₃, O(CH₂)₂SCH₃, O(CH₂)₂ON(CH₃)₂, O(CH₂)₂O(CH₂)₂N(CH₃)₂, and OCH₂C(=O)-N(H)CH₃("NMA"). In certain embodiments, the 2'-substituted non-bicyclic modified nucleoside comprises a sugar moiety comprising a non-bridging 2'-substituent selected from the group consisting of: F (2'-F), OCH₃ (2'-OMe), and OCH₂CH₂OCH₃ (2'-MOE).

In certain embodiments, the modified furanosyl sugar moiety and the nucleoside incorporating such a modified furanosyl sugar moiety are further defined by isomeric configuration. For example, a 2'-deoxyfuranosyl sugar moiety may be in any of seven isomeric configurations other than the naturally occurring β-D-deoxyribosyl configuration. Such modified sugar moieties are set forth in, for example, WO 2019/157531, which is incorporated herein by reference. A 2'-modified sugar moiety has an additional stereocenter at the 2'-position relative to the 2'-deoxyfuranosyl sugar moiety; therefore, such sugar moieties have a total of sixteen possible isomeric configurations. Unless otherwise specified, the 2'-modified sugar moieties described herein are in the β-D-ribosyl isomeric configuration.

In some embodiments, the antisense oligonucleotide comprises at least one sugar surrogate. In certain such embodiments, the oxygen atom of the sugar moiety is replaced by, for example, a sulfur, carbon, or nitrogen atom. In certain such embodiments, such modified sugar moieties also comprise bridging and/or non-bridging substituents as set forth herein. For example, certain sugar surrogates comprise a 4'-sulfur atom and substituents at the 2'-position (see, e.g., Bhat et al., U.S. 7,875,733 and Bhat et al., U.S. 7,939,677) and/or the 5'-position.

In certain embodiments, the sugar surrogate comprises a ring having other than five atoms. For example, in certain embodiments, the sugar surrogate comprises a six-membered tetrahydropyran ("THP"). Such tetrahydropyrans may be further modified or substituted. Nucleosides comprising such modified tetrahydropyrans include, but are not limited to, hexitol nucleic acid ("HNA"), anitol nucleic acid ("ANA"), mannitol nucleic acid ("MNA") (see, e.g., Leumann, C. J. Bioorg. & Med. Chem. 2002, 10, 841-854), and fluoro HNA (F-HNA, also referred to as F-THP or 3'-fluoro tetrahydropyran).

In certain embodiments, the sugar surrogate comprises a ring having more than five atoms and more than one heteroatom. For example, nucleosides comprising a morpholino sugar moiety and their use in oligonucleotides have been reported. In certain embodiments, the sugar surrogate comprises an acyclic moiety. Examples of nucleosides and oligonucleotides comprising such acyclic sugar surrogates include, but are not limited to, peptide nucleic acid ("PNA"), acyclic butyl nucleic acid (see, e.g., Kumar et al., Org. Biomol. Chem., 2013, 11, 5853-5865), and the nucleosides and oligonucleotides set forth in Manoharan et al., WO 2011/133876. By introducing a modified nucleobase having a cationic lipid or its equivalent covalently linked thereto, PNA can be rendered highly permeable to mammalian cell membranes (see, e.g., WO 2019/022434 and WO 2009/113828). In preferred embodiments, the sugar surrogate is selected from POM, PNA, THP, and F-HNA.

In some embodiments, the antisense oligonucleotide comprises one or more nucleosides comprising an unmodified nucleobase. In certain embodiments, the antisense oligonucleotide comprises one or more nucleosides comprising a modified nucleobase. In certain embodiments, the antisense oligonucleotide comprises one or more nucleosides that do not comprise a nucleobase, referred to as abasic nucleosides.

In certain embodiments, the modified nucleobase is selected from: 5-substituted pyrimidines (e.g., 5-methylcytosine, m5C), 6-azapyrimidines, alkyl- or alkynyl-substituted pyrimidines, alkyl-substituted purines, and N-2, N-6, and O-6 substituted purines. In certain embodiments, the modified nucleobase is selected from: 2-aminopropyladenine, 5-hydroxymethylcytosine, xanthine, hypoxanthine, 2-aminoadenine, 6-N-methylguanine, 6-N-methyladenine, 2-propyladenine, 2-thiouracil, 2-thiothymine, and 2-thiocytosine, 5-propynyl (-C≡C-CH₃) uracil, 5-propynylcytosine, 6-azouracil, 6-azocytosine, 6-azothymine, 5-ribosyluracil (pseudouracil), 4-thiouracil; 8-halo-, 8-amino-, 8-thiol-, 8-thioalkyl-, 8-hydroxy-, 8-aza,- and other 8-substituted purines; 5-halo-, particularly 5-bromo-, 5-trifluoromethyl-, 5-halouracil, and 5-halocytosine; 7-methylguanine, 7-methyladenine, 2-F-adenine, 2-aminoadenine, 7-deazaguanine, 7-deazaadenine, 3-deazaguanine, 3-deazaadenine, 6-N-benzoyladenine, 2-N-isobutyrylguanine, 5-methylcytosine, 4-N-benzoylcytosine, 4-N-benzoyluracil, 5-methyl-4-N-benzoylcytosine, 5-methyl-4-N-benzoyluracil, universal bases, hydrophobic bases, promiscuous bases, size-expanded bases, and fluorinated bases. Other modified nucleobases include tricyclic pyrimidines, such as 1,3-diazaphenoxazine-2-one, 1,3-diazaphenothiazine-2-one, and 9-(2-aminoethoxy)-1,3-diazaphenoxazine-2-one (G-clamp). Modified nucleobases may also include those in which the purine or pyrimidine base is replaced by other heterocycles, such as 7-deaza-adenine, 7-deazaguanosine, 2-aminopyridine, and 2-pyridone. In preferred embodiments, the antisense oligonucleotide comprises one or more 5-methylcytosines (m5C).

In some embodiments, the modified nucleobase may be a nucleobase having a cationic lipid covalently linked thereto. By introducing a modified nucleobase having a cationic lipid or an equivalent thereof covalently linked thereto, the antisense oligonucleotide can be rendered highly permeable to mammalian cell membranes. Suitable cationic lipid-linked modifications covalently attached thereto may be those described in WO 2019022434A1, which is incorporated herein by reference in its entirety.

In some embodiments, the antisense oligonucleotide comprises at least one modified internucleoside linkage. In preferred embodiments, every internucleoside linkage of the antisense oligonucleotide is a modified internucleoside linkage. In certain embodiments, any internucleoside linkage may be used to link the nucleosides of the antisense oligonucleotide together. Two main classes of internucleoside linking groups are defined by the presence or absence of a phosphorus atom. Representative phosphorus-containing internucleoside linkages include, but are not limited to, phosphodiester, which contains a phosphodiester bond ("P(O₂)=O") (also referred to as an unmodified linkage or naturally occurring linkage); phosphotriester; methylphosphonate; phosphoramidate (PN); phosphorothioate ("P(O₂)=S", PS); and phosphorodithioate ("HS-P=S"). Phosphoramidate (PN) may have the structural formula - N(R)P(=X)(OH)O-, -OP(=X)(OH)N(R)-, -O-P(NR)(=X)O-, - N(SO2R)P(=X)(OH)O-, - OP(=X)(OH)N(SO₂R)-, or -O-P(NSO₂R)(=X)O- ("MsPA"), wherein X is O or S, R may be optionally substituted alkyl, aryl, heteroaryl, or heterocycloalkyl; or NR may be an optionally substituted cyclic guanidinyl moiety, an optionally substituted triazolyl group, or a Tmg group. Further PN structures can be found in WO 2023220744, WO 2019/032612, and WO 2021/030778, which are incorporated herein by reference in their entirety.

Representative phosphorus-free internucleoside linking groups include, but are not limited to, methylenemethylimino (-CH₂-N(CH₃)-O-CH₂-), thiodiester, thiocarbamate (-O-C( = O)(NH)-S-); siloxane (-O-SiH₂-O-); and N,N'-dimethylhydrazine (-CH₂-N(CH₃)-N(CH₃)-). Modified internucleoside linkages can be used to alter, and typically increase, the nuclease resistance of oligonucleotides compared to naturally occurring phosphodiester internucleoside linkages. In certain embodiments, internucleoside linkages having a chiral atom can be prepared as racemic mixtures or as separated enantiomers. Methods for preparing phosphorus-containing and phosphorus-free internucleoside linkages are well known to those skilled in the art.

Representative internucleoside linkages having a chiral center include, but are not limited to, alkylphosphonates and phosphorothioates. Antisense oligonucleotides comprising internucleoside linkages having a chiral center may be prepared as a population of antisense oligonucleotides comprising stereorandom internucleoside linkages, or as a population of antisense oligonucleotides comprising phosphorothioate internucleoside linkages in a specific stereochemical configuration. In certain embodiments, a population of antisense oligonucleotides comprises phosphorothioate internucleoside linkages, wherein all of said phosphorothioate internucleoside linkages are stereorandom. Such antisense oligonucleotides can be generated using synthetic methods that randomly select the stereochemical configuration of each phosphorothioate internucleoside linkage. Nevertheless, as is well understood by those skilled in the art, each individual phosphorothioate linkage of each individual oligonucleotide molecule has a defined stereochemical configuration. In certain embodiments, the population of antisense oligonucleotides is enriched for antisense oligonucleotides comprising one or more specific phosphorothioate internucleoside linkages in a specific, independently selected stereochemical configuration. In certain embodiments, at least 65% of the molecules in the population have the specific phosphorothioate internucleoside linkage in a specific configuration. In certain embodiments, at least 70% of the molecules in the population have the specific phosphorothioate internucleoside linkage in a specific configuration. In certain embodiments, at least 80% of the molecules in the population have the specific phosphorothioate internucleoside linkage in a specific configuration. In certain embodiments, at least 90% of the molecules in the population have the specific phosphorothioate internucleoside linkage in a specific configuration. In certain embodiments, at least 99% of the molecules in the population have the specific phosphorothioate internucleoside linkage in a specific configuration. Such chirally enriched populations of antisense oligonucleotides can be generated using synthetic methods known in the art, such as those set forth in Oka et al., JACS 125, 8307 (2003); Wan et al., Nuc. Acid. Res. 42, 13456 (2014); and WO 2017/015555. In certain embodiments, the population of antisense oligonucleotides is enriched for antisense oligonucleotides having at least one indicated phosphorothioate linkage in the (Sp) configuration. In certain embodiments, the population of antisense oligonucleotides is enriched for antisense oligonucleotides having at least one phosphorothioate linkage in the (Rp) configuration.

In some embodiments, the antisense oligonucleotide comprises a gapmer. Said gapmer is defined by two external regions or "wings" and a central or internal region or "gap." The three regions of the gapmer motif (5'-wing, gap, and 3'-wing) form a contiguous sequence of nucleosides, wherein at least some sugar moieties of the nucleosides of each wing are different from at least some sugar moieties of the nucleosides of the gap. Specifically, at least the sugar moiety of the nucleoside in each wing that is closest to the gap (the 3'-most nucleoside of the 5'-wing and the 5'-most nucleoside of the 3'-wing) is different from the sugar moiety of the adjacent gap nucleoside, thereby defining the boundary between the wing and the gap (i.e., the wing/gap junction). In certain embodiments, the sugar moieties within the gap are identical to each other. In certain embodiments, the gap comprises one or more nucleosides having a sugar moiety that is different from the sugar moiety of one or more other nucleosides of the gap. In certain embodiments, the sugar motifs of the two wings are identical to each other (symmetric gapmer). In certain embodiments, the sugar motif of the 5'-wing is different from the sugar motif of the 3'-wing (asymmetric gapmer).

In some embodiments, the gapmer comprises: (a) a 5' region consisting of 1-6 linked 5' region nucleosides; (b) a central region consisting of 6-10 linked central region nucleosides; and (c) a 3' region consisting of 1-6 linked 3' region nucleosides; wherein each of the nucleosides in the 5' region and the 3' region comprises a sugar moiety selected from 2'-MOE, LNA, and cEt modifications, and at least 6 of the nucleosides in the central region comprise a 2'-deoxy sugar moiety.

In some embodiments, the gapmer has a sugar motif (5' to 3') selected from: eekddddddddddkke, ekkddddddddddkke, kkkdyddddddddkkk, kkkddydddddddkkk, kkkdddyddddddkkk, kkkddddddddddkkk, or eeeeeddddddddddeeeee; wherein e represents a 2'-MOE sugar moiety, k represents a cEt sugar moiety, d represents a 2'-deoxy sugar moiety, and y represents a 2'-OMe sugar moiety.

In preferred embodiments, the gapmer has the sugar motif eeeeeeddddddddddeeeee (5' to 3'), wherein e represents a 2'-MOE sugar moiety and d represents a 2'-deoxy sugar moiety.

In preferred embodiments, the internucleoside linkages of the gapmer are PS. In such embodiments, the gapmer comprises or consists of a nucleotide sequence as set forth in any one of SEQ ID NOs: 21 to 38, or a nucleotide sequence having 1, 2, or 3 nucleotide differences therefrom. In one embodiment, the gapmer comprises or consists of a nucleotide sequence as set forth in SEQ ID NO: 21, or a nucleotide sequence having 1, 2, or 3 nucleotide differences therefrom. In one embodiment, the gapmer comprises or consists of a nucleotide sequence as set forth in SEQ ID NO: 22, or a nucleotide sequence having 1, 2, or 3 nucleotide differences therefrom. In one embodiment, the gapmer comprises or consists of a nucleotide sequence as set forth in SEQ ID NO: 23, or a nucleotide sequence having 1, 2, or 3 nucleotide differences therefrom. In one embodiment, the gapmer comprises or consists of a nucleotide sequence as set forth in SEQ ID NO: 24, or a nucleotide sequence having 1, 2, or 3 nucleotide differences therefrom. In one embodiment, the gapmer comprises or consists of a nucleotide sequence as set forth in SEQ ID NO: 25, or a nucleotide sequence having 1, 2, or 3 nucleotide differences therefrom. In one embodiment, the gapmer comprises or consists of a nucleotide sequence as set forth in SEQ ID NO: 26, or a nucleotide sequence having 1, 2, or 3 nucleotide differences therefrom. In one embodiment, the gapmer comprises or consists of a nucleotide sequence as set forth in SEQ ID NO: 27, or a nucleotide sequence having 1, 2, or 3 nucleotide differences therefrom. In one embodiment, the gapmer comprises or consists of a nucleotide sequence as set forth in SEQ ID NO: 28, or a nucleotide sequence having 1, 2, or 3 nucleotide differences therefrom. In one embodiment, the gapmer comprises or consists of a nucleotide sequence as set forth in SEQ ID NO: 29, or a nucleotide sequence having 1, 2, or 3 nucleotide differences therefrom. In one embodiment, the gapmer comprises or consists of a nucleotide sequence as set forth in SEQ ID NO: 30, or a nucleotide sequence having 1, 2, or 3 nucleotide differences therefrom. In one embodiment, the gapmer comprises or consists of a nucleotide sequence as set forth in SEQ ID NO: 31, or a nucleotide sequence having 1, 2, or 3 nucleotide differences therefrom. In one embodiment, the gapmer comprises or consists of a nucleotide sequence as set forth in SEQ ID NO: 32, or a nucleotide sequence having 1, 2, or 3 nucleotide differences therefrom. In one embodiment, the gapmer comprises or consists of a nucleotide sequence as set forth in SEQ ID NO: 33, or a nucleotide sequence having 1, 2, or 3 nucleotide differences therefrom. In one embodiment, the gapmer comprises or consists of a nucleotide sequence as set forth in SEQ ID NO: 34, or a nucleotide sequence having 1, 2, or 3 nucleotide differences therefrom. In one embodiment, the gapmer comprises or consists of a nucleotide sequence as set forth in SEQ ID NO: 35, or a nucleotide sequence having 1, 2, or 3 nucleotide differences therefrom. In one embodiment, the gapmer comprises or consists of a nucleotide sequence as set forth in SEQ ID NO: 36, or a nucleotide sequence having 1, 2, or 3 nucleotide differences therefrom. In one embodiment, the gapmer comprises or consists of a nucleotide sequence as set forth in SEQ ID NO: 37, or a nucleotide sequence having 1, 2, or 3 nucleotide differences therefrom. In one embodiment, the gapmer comprises or consists of a nucleotide sequence as set forth in SEQ ID NO: 38, or a nucleotide sequence having 1, 2, or 3 nucleotide differences therefrom.

### RNAi Agents For Inhibiting SCN10A Gene Expression

One aspect of the present invention provides an RNAi agent for inhibiting SCN10A gene expression, comprising a sense strand and an antisense strand that form a complementary duplex region, wherein the antisense strand comprises at least 15 (e.g., 15, 16, 17, 18, 19, 20, or 21) contiguous nucleotides of a nucleotide sequence selected from SEQ ID NOs: 59 to 78 and nucleotide sequences having 1, 2, or 3 nucleotide differences each therefrom.

In some embodiments, the present invention provides an RNAi agent for inhibiting SCN10A gene expression, comprising a sense strand and an antisense strand that form a complementary duplex region, wherein the antisense strand comprises at least 15 (e.g., 15, 16, 17, 18, 19, 20, or 21) contiguous nucleotides selected from any one of the nucleotide sequences of SEQ ID NOs: 59 to 78.

In some embodiments, the present invention provides an RNAi agent for inhibiting SCN10A gene expression, comprising a sense strand and an antisense strand that form a complementary duplex region, wherein the antisense strand comprises at least 15 (e.g., 15, 16, 17, 18, 19, 20, or 21) contiguous nucleotides selected from a nucleotide sequence that differs from any one of the nucleotide sequences of SEQ ID NOs: 59 to 78 by 1, 2, or 3 nucleotides.

In the present invention, when referring to 1, 2, or 3 nucleotide differences, the 1, 2, or 3 nucleotide differences may be on the sense strand and/or antisense strand outside the duplex region. In some embodiments, the 1, 2, or 3 nucleotide differences are on the sense strand and/or antisense strand within the duplex region. In some embodiments, a portion of the 1, 2, or 3 nucleotide differences are on the sense strand and/or antisense strand within the duplex region, and another portion are on the sense strand and/or antisense strand outside the duplex region. For the antisense strand, in some embodiments, the 1, 2, or 3 nucleotide differences are located at the extreme 3' end or extreme 5' end of the antisense strand. In some embodiments, the 1, 2, or 3 nucleotide differences are located between the extreme 3' end and extreme 5' end of the antisense strand. In some embodiments, 1 or 2 of the 1, 2, or 3 nucleotide differences are located at the extreme 3' end or extreme 5' end of the antisense strand, and the other 1 or 2 are located between the extreme 3' end and extreme 5' end of the antisense strand. For the sense strand, in some embodiments, the 1, 2, or 3 nucleotide differences are located at the extreme 3' end or extreme 5' end of the sense strand. In some embodiments, the 1, 2, or 3 nucleotide differences are located between the extreme 3' end and extreme 5' end of the sense strand. In some embodiments, 1 or 2 of the 1, 2, or 3 nucleotide differences are located at the extreme 3' end or extreme 5' end of the sense strand, and the other 1 or 2 are located between the extreme 3' end and extreme 5' end of the sense strand.

In some embodiments, the present invention provides an RNAi agent for inhibiting SCN10A gene expression, comprising a sense strand and an antisense strand that form a complementary duplex region, wherein the antisense strand comprises at least 15 (e.g., 15, 16, 17, 18, 19, 20, or 21) contiguous nucleotides of a nucleotide sequence having 1 nucleotide difference from any nucleotide sequence selected from SEQ ID NOs: 59 to 78, the 1 nucleotide difference being located at the extreme 3' end of the antisense strand. In some embodiments, the present invention provides an RNAi agent for inhibiting SCN10A gene expression, comprising a sense strand and an antisense strand that form a complementary duplex region, wherein the antisense strand comprises at least 15 (e.g., 15, 16, 17, 18, 19, 20, or 21) contiguous nucleotides of a nucleotide sequence having 2 nucleotide differences from any nucleotide sequence selected from SEQ ID NOs: 59 to 78, the 2 nucleotide differences being located consecutively at the extreme 3' end of the antisense strand.

In some embodiments, the present invention provides an RNAi agent for inhibiting SCN10A gene expression, comprising a sense strand and an antisense strand that form a complementary duplex region, wherein the antisense strand comprises no more than 23 nucleotides in length and comprises at least 15 (e.g., 15, 16, 17, 18, 19, 20, or 21) contiguous nucleotides of any nucleotide sequence selected from SEQ ID NOs: 59 to 78.

In some embodiments, the present invention provides an RNAi agent for inhibiting SCN10A gene expression, comprising a sense strand and an antisense strand that form a complementary duplex region, wherein the antisense strand comprises no more than 23 nucleotides in length and comprises at least 15 (e.g., 15, 16, 17, 18, 19, 20, or 21) contiguous nucleotides of a nucleotide sequence having 1, 2, or 3 nucleotide differences from any nucleotide sequence selected from SEQ ID NOs: 59 to 78.

One aspect of the present invention provides an RNAi agent for inhibiting SCN10A gene expression, comprising an antisense strand, wherein the antisense strand comprises at least 15 (e.g., 15, 16, 17, 18, 19, 20, or 21) contiguous nucleotides of a nucleotide sequence selected from SEQ ID NOs: 59 to 78 and nucleotide sequences having 1, 2, or 3 nucleotide differences therefrom.

In some embodiments, the present invention provides an RNAi agent for inhibiting SCN10A gene expression, comprising an antisense strand, wherein the antisense strand comprises at least 15 (e.g., 15, 16, 17, 18, 19, 20, or 21) contiguous nucleotides of any nucleotide sequence selected from SEQ ID NOs: 59 to 78.

In some embodiments, the present invention provides an RNAi agent for inhibiting SCN10A gene expression, comprising an antisense strand, wherein the antisense strand comprises at least 15 (e.g., 15, 16, 17, 18, 19, 20, or 21) contiguous nucleotides of a nucleotide sequence having 1, 2, or 3 nucleotide differences from any nucleotide sequence selected from SEQ ID NOs: 59 to 78.

In some embodiments, the length of the duplex region is about 17 to about 23 base pairs. For example, suitable lengths for the duplex region are about 17 to about 22 base pairs, about 17 to about 21 base pairs, about 17 to about 20 base pairs, about 17 to about 19 base pairs, about 17 to about 18 base pairs, about 18 to about 23 base pairs, about 18 to about 22 base pairs, about 18 to about 21 base pairs, about 18 to about 20 base pairs, about 19 to 23 base pairs, about 19 to 22 base pairs, about 19 to 21 base pairs, about 20 to 23 base pairs, about 20 to 22 base pairs, or about 21 to 23 base pairs. In certain embodiments, the length of the duplex region is about 18 to about 21 base pairs. In other embodiments, the length of the duplex region is about 19 base pairs.

Accordingly, in some embodiments of the present invention, an RNAi agent for inhibiting SCN10A gene expression is provided, comprising a sense strand and an antisense strand that form a complementary duplex region, the length of the duplex region being about 17 to about 23 base pairs, and wherein the antisense strand is no more than 23 nucleotides in length and comprises at least 15 contiguous nucleotides of a nucleotide sequence selected from SEQ ID NOs: 59 to 78 and nucleotide sequences having 1 to 3 nucleotide differences therefrom.

In some embodiments of the present invention, an RNAi agent for inhibiting SCN10A gene expression is provided, comprising a sense strand and an antisense strand that form a complementary duplex region, the length of the duplex region being about 18 to about 21 base pairs, and wherein the antisense strand is no more than 23 nucleotides in length and comprises at least 15 contiguous nucleotides of a nucleotide sequence selected from SEQ ID NOs: 59 to 78 and nucleotide sequences having 1 to 3 nucleotide differences therefrom.

In some embodiments of the present invention, an RNAi agent for inhibiting SCN10A gene expression is provided, comprising a sense strand and an antisense strand that form a complementary duplex region, the length of the duplex region being about 19 base pairs, and wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 59 to 78 and nucleotide sequences having 1, 2, or 3 nucleotide differences therefrom.

In some embodiments, the sense and antisense strands of the RNAi agent of the invention each independently have a length of about 17 to about 23 nucleotides, e.g., about 18 to about 23 nucleotides, about 19 to about 23 nucleotides, about 20 to about 23 nucleotides, about 21 to about 23 nucleotides, about 17 to about 22 nucleotides, about 17 to about 21 nucleotides, about 17 to about 20 nucleotides, about 17 to about 19 nucleotides, about 18 to about 22 nucleotides, about 18 to about 21 nucleotides, about 18 to about 20 nucleotides, about 19 to about 22 nucleotides, about 19 to about 21 nucleotides, or about 20 to about 22 nucleotides. In certain embodiments, the sense and antisense strands each independently have a length of about 17, about 18, about 19, about 20, about 21, about 22, or about 23 nucleotides.

In some embodiments, the sense strand and antisense strand are of identical length but form a duplex region shorter than the full length of the strands, such that the RNAi agent for inhibiting SCN10A gene expression comprises two nucleotide overhangs. For example, in one embodiment, the RNAi agent for inhibiting SCN10A gene expression comprises (i) a sense strand and an antisense strand each having a length of 21 nucleotides, (ii) a duplex region having a length of 19 base pairs, and (iii) a single-nucleotide overhang at each of 3'-end of the sense strand and 3'-end of the antisense strand. In another embodiment, the RNAi agent for inhibiting SCN10A gene expression comprises (i) a sense strand and an antisense strand each having a length of 23 nucleotides, (ii) a duplex region having a length of 21 base pairs, and (iii) a single-nucleotide overhang at each of 3'-end of the sense strand and 3'-end of the antisense strand.

In other embodiments, the sense strand and antisense strand are of identical length and form a duplex region along their entire length, such that no nucleotide overhangs are present at either end of the duplex molecule. In one such embodiment, the RNAi agent for inhibiting SCN10A gene expression is blunt-ended and comprises (i) a sense strand and an antisense strand each having a length of 21 nucleotides, and (ii) a duplex region having a length of 21 base pairs. In another such embodiment, the RNAi agent for inhibiting SCN10A gene expression is blunt-ended and comprises (i) a sense strand and an antisense strand each having a length of 23 nucleotides, and (ii) a duplex region having a length of 23 base pairs. In another such embodiment, the RNAi agent for inhibiting SCN10A gene expression is blunt-ended and comprises (i) a sense strand and an antisense strand each having a length of 19 nucleotides, and (ii) a duplex region having a length of 19 base pairs.

In other embodiments, the sense strand or antisense strand is longer than the other strand, and the two strands form a duplex region of length equal to that of the shorter strand, such that the RNAi agent for inhibiting SCN10A gene expression comprises at least one nucleotide overhang. For example, in some embodiments, the sense strand is 1 to 4 nucleotides longer than the antisense strand, and the duplex region formed by both strands equals the length of the antisense strand, such that the sense strand forms an overhang comprising 1 to 4 unpaired nucleotides. In other embodiments, the antisense strand is 1 to 4 nucleotides longer than the sense strand, and the duplex region formed by both strands equals the length of the sense strand, such that the antisense strand forms an overhang comprising 1 to 4 unpaired nucleotides. In some embodiments, the nucleotide overhang has a length of 1, 2, 3, or 4 nucleotides. In a specific embodiment, the overhang comprises 2 nucleotides. In certain embodiments, the overhang comprises a single nucleotide.

The overhanging nucleotides may be ribonucleotides or modified nucleotides as described herein. In some embodiments, the overhanging nucleotides are 2'-modified nucleotides (e.g., 2'-fluoro-modified nucleotides, 2'-O-methyl-modified nucleotides) or combinations thereof. For example, in one embodiment, the overhanging nucleotides are deoxyribonucleotides, such as deoxythymidine. In another embodiment, the overhanging nucleotides are selected from: 2'-O-methyl-modified nucleotides, 2'-fluoro-modified nucleotides, 2'-methoxyethyl-modified nucleotides, abasic nucleotides, inverted abasic nucleotides, inverted nucleotides, or combinations thereof. In other embodiments, the overhang comprises a 5'-uridine-uridine-3' (5'-UU-3') dinucleotide. In such embodiments, the UU dinucleotide may comprise ribonucleotides or modified nucleotides, such as 2'-modified nucleotides. In other embodiments, the overhang comprises a 5'-deoxythymidine-deoxythymidine-3' (5'-dTdT-3') dinucleotide. When a nucleotide overhang is present on the antisense strand, nucleotides in the overhang may be complementary to the target gene sequence, may form mismatches with the target gene sequence, or may comprise other sequences (such as UU, TT, AA, GG, etc.).

The nucleotide overhang may be located at the 5'-end or 3'-end of either strand or both strands. For example, in one embodiment, the RNAi agent for inhibiting SCN10A gene expression comprises nucleotide overhangs at both 5'-end and 3'-end of the antisense strand. In another embodiment, the RNAi agent for inhibiting SCN10A gene expression comprises nucleotide overhangs at both 5'-end and 3'-end of the sense strand. In some embodiments, the RNAi agent for inhibiting SCN10A gene expression comprises nucleotide overhangs at the 5'-end of the sense strand and the 5'-end of the antisense strand. In other embodiments, the RNAi agent for inhibiting SCN10A gene expression comprises nucleotide overhangs at the 3'-end of the sense strand and the 3'-end of the antisense strand. In some embodiments, the RNAi agent for inhibiting SCN10A gene expression comprises only a nucleotide overhang at the 5'-end of the sense strand. In some embodiments, the RNAi agent for inhibiting SCN10A gene expression comprises only a nucleotide overhang at the 3'-end of the sense strand. In some embodiments, the RNAi agent for inhibiting SCN10A gene expression comprises only a nucleotide overhang at the 3'-end of the antisense strand. In some embodiments, the RNAi agent for inhibiting SCN10A gene expression comprises only a nucleotide overhang at the 5'-end of the antisense strand. In some embodiments, the RNAi agent for inhibiting SCN10A gene expression comprises only a nucleotide overhang at the 5'-end of the sense strand.

The RNAi agent for inhibiting SCN10A gene expression may comprise a nucleotide overhang at one end and a blunt end at the opposite end of the double-stranded RNA molecule. "Blunt end" means that the sense strand and antisense strand are fully base-paired at the molecular terminus, with no unpaired nucleotides extending beyond the duplex region. In some embodiments, the RNAi agent for inhibiting SCN10A gene expression comprises a nucleotide overhang at the 3'-end of the sense strand, and blunt ends at the 5'-end of the sense strand and the 3'-end of the antisense strand. In other embodiments, the RNAi agent for inhibiting SCN10A gene expression comprises a nucleotide overhang at the 3'-end of the antisense strand, and blunt ends at the 5'-end of the antisense strand and the 3'-end of the sense strand.

Specifically, by way of example, in one embodiment, the RNAi agent for inhibiting SCN10A gene expression comprises: (i) a sense strand of 19 nucleotides in length, (ii) an antisense strand of 21 nucleotides in length, wherein the strands form a duplex region equal to the length of the sense strand. In another embodiment, the RNAi agent for inhibiting SCN10A gene expression comprises: (i) a sense strand of 21 nucleotides in length, (ii) an antisense strand of 23 nucleotides in length, wherein the strands form a duplex region equal to the length of the sense strand.

In some embodiments, an RNAi agent for inhibiting SCN10A gene expression in cells is provided, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 59 to 78 and nucleotide sequences having 1, 2, or 3 nucleotide differences therefrom, and wherein the RNAi agent comprises one overhang and one blunt end, the overhang preferably having 2 unpaired nucleotides.

In some embodiments, an RNAi agent for inhibiting SCN10A gene expression in cells is provided, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 59 to 78 and nucleotide sequences having 1, 2, or 3 nucleotide differences therefrom, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting SCN10A gene expression in cells is provided, comprising a sense strand and an antisense strand that form a duplex region of 19 base pairs in length, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 59 to 78 and nucleotide sequences having 1, 2, or 3 nucleotide differences therefrom, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting SCN10A gene expression in cells is provided, comprising a sense strand and an antisense strand that form a duplex region of 19 base pairs in length, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 59 to 78 and nucleotide sequences having 1, 2, or 3 nucleotide differences therefrom, wherein the sense strand is 19 to 21 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, an RNAi agent for inhibiting SCN10A gene expression in cells is provided, comprising a sense strand and an antisense strand that form a duplex region of 19 base pairs in length, wherein the antisense strand is 21 nucleotides in length and consists of at least 15 (e.g., 15, 16, 17, 18, 19, 20, or 21) contiguous nucleotides of a nucleotide sequence selected from SEQ ID NOs: 59 to 78 and nucleotide sequences having 1, 2, or 3 nucleotide differences therefrom, wherein the sense strand is 19 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In some embodiments, the present invention provides an RNAi agent for inhibiting SCN10A gene expression in cells, comprising a sense strand and an antisense strand that form a duplex region of 19 base pairs in length, wherein the antisense strand is 21 nucleotides in length and consists of any nucleotide sequence selected from SEQ ID NOs: 59 to 78, wherein the sense strand is 19 nucleotides in length, and wherein the RNAi agent comprises one overhang and one blunt end, the overhang preferably having 2 unpaired nucleotides, wherein the overhang is formed at 3'-end of the antisense strand, and the blunt end is formed at 3'-end of the sense strand and 5'-end of the antisense strand.

In preferred embodiments, the present invention provides an RNAi agent for inhibiting SCN10A gene expression in cells, comprising a sense strand and an antisense strand that form a duplex region, wherein the antisense strand comprises at least 15 (e.g., 15, 16, 17, 18, 19, 20, or 21) contiguous nucleotides of a nucleotide sequence selected from SEQ ID NOs: 59 to 78 and nucleotide sequences having 1, 2, or 3 nucleotide differences therefrom, and wherein the sense strand comprises at least 15 (e.g., 15, 16, 17, 18, or 19) contiguous nucleotides of any nucleotide sequence selected from SEQ ID NOs: 39 to 58 and nucleotide sequences having 1, 2, or 3 nucleotide differences therefrom.

In preferred embodiments, the present invention provides an RNAi agent for inhibiting SCN10A gene expression in cells, comprising a sense strand and an antisense strand that form a duplex region, wherein the antisense strand is no more than 23 nucleotides in length and comprises at least 15 (e.g., 15, 16, 17, 18, 19, 20, or 21) contiguous nucleotides of a nucleotide sequence selected from SEQ ID NOs: 59 to 78 and nucleotide sequences having 1, 2, or 3 nucleotide differences therefrom, and wherein the sense strand is no more than 21 nucleotides in length and comprises at least 15 (e.g., 15, 16, 17, 18, or 19) contiguous nucleotides of any nucleotide sequence selected from SEQ ID NOs: 39 to 58 and nucleotide sequences having 1, 2, or 3 nucleotide differences therefrom.

In preferred embodiments, the present invention provides an RNAi agent for inhibiting SCN10A gene expression in cells, comprising a sense strand and an antisense strand that form a duplex region, wherein the antisense strand is no more than 23 nucleotides in length and comprises any nucleotide sequence selected from SEQ ID NOs: 59 to 78, and wherein the sense strand is no more than 21 nucleotides in length and comprises any nucleotide sequence selected from SEQ ID NOs: 39 to 58.

In preferred embodiments, the present invention provides an RNAi agent for inhibiting SCN10A gene expression in a cell, comprising a sense strand and an antisense strand that form a duplex region, wherein:
the sense strand comprises or consists of a sequence set forth in SEQ ID NO:39 or a nucleotide sequence having 1, 2, or 3 nucleotide differences therefrom, and the antisense strand comprises or consists of a sequence set forth in SEQ ID NO:59 or a nucleotide sequence having 1, 2, or 3 nucleotide differences therefrom;
the sense strand comprises or consists of a sequence set forth in SEQ ID NO:40 or a nucleotide sequence having 1, 2, or 3 nucleotide differences therefrom, and the antisense strand comprises or consists of a sequence set forth in SEQ ID NO:60 or a nucleotide sequence having 1, 2, or 3 nucleotide differences therefrom;
the sense strand comprises or consists of a sequence set forth in SEQ ID NO:41 or a nucleotide sequence having 1, 2, or 3 nucleotide differences therefrom, and the antisense strand comprises or consists of a sequence set forth in SEQ ID NO:61 or a nucleotide sequence having 1, 2, or 3 nucleotide differences therefrom;
the sense strand comprises or consists of a sequence set forth in SEQ ID NO:42 or a nucleotide sequence having 1, 2, or 3 nucleotide differences therefrom, and the antisense strand comprises or consists of a sequence set forth in SEQ ID NO:62 or a nucleotide sequence having 1, 2, or 3 nucleotide differences therefrom;
the sense strand comprises or consists of a sequence set forth in SEQ ID NO:43 or a nucleotide sequence having 1, 2, or 3 nucleotide differences therefrom, and the antisense strand comprises or consists of a sequence set forth in SEQ ID NO:63 or a nucleotide sequence having 1, 2, or 3 nucleotide differences therefrom;
the sense strand comprises or consists of a sequence set forth in SEQ ID NO:44 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom;
the sense strand comprises or consists of a sequence set forth in SEQ ID NO:45 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom;
the sense strand comprises or consists of a sequence set forth in SEQ ID NO:46 or a nucleotide sequence having 1, 2, or 3 nucleotide differences therefrom, and the antisense strand comprises or consists of a sequence set forth in SEQ ID NO:66 or a nucleotide sequence having 1, 2, or 3 nucleotide differences therefrom;
the sense strand comprises or consists of a sequence set forth in SEQ ID NO:47 or a nucleotide sequence having 1, 2, or 3 nucleotide differences therefrom, and the antisense strand comprises or consists of a sequence set forth in SEQ ID NO:67 or a nucleotide sequence having 1, 2, or 3 nucleotide differences therefrom;
the sense strand comprises or consists of a sequence set forth in SEQ ID NO:48 or a nucleotide sequence having 1, 2, or 3 nucleotide differences therefrom, and the antisense strand comprises or consists of a sequence set forth in SEQ ID NO:68 or a nucleotide sequence having 1, 2, or 3 nucleotide differences therefrom;
the sense strand comprises or consists of a sequence set forth in SEQ ID NO:49 or a nucleotide sequence having 1, 2, or 3 nucleotide differences therefrom, and the antisense strand comprises or consists of a sequence set forth in SEQ ID NO:69 or a nucleotide sequence having 1, 2, or 3 nucleotide differences therefrom;
the sense strand comprises or consists of a sequence set forth in SEQ ID NO:50 or a nucleotide sequence having 1, 2, or 3 nucleotide differences therefrom, and the antisense strand comprises or consists of a sequence set forth in SEQ ID NO:70 or a nucleotide sequence having 1, 2, or 3 nucleotide differences therefrom;
the sense strand comprises or consists of a sequence set forth in SEQ ID NO:51 or a nucleotide sequence having 1, 2, or 3 nucleotide differences therefrom, and the antisense strand comprises or consists of a sequence set forth in SEQ ID NO:71 or a nucleotide sequence having 1, 2, or 3 nucleotide differences therefrom;
the sense strand comprises or consists of a sequence set forth in SEQ ID NO:52 or a nucleotide sequence having 1, 2, or 3 nucleotide differences therefrom, and the antisense strand comprises or consists of a sequence set forth in SEQ ID NO:72 or a nucleotide sequence having 1, 2, or 3 nucleotide differences therefrom;
the sense strand comprises or consists of a sequence set forth in SEQ ID NO:53 or a nucleotide sequence having 1, 2, or 3 nucleotide differences therefrom, and the antisense strand comprises or consists of a sequence set forth in SEQ ID NO:73 or a nucleotide sequence having 1, 2, or 3 nucleotide differences therefrom;
the sense strand comprises or consists of a sequence set forth in SEQ ID NO:54 or a nucleotide sequence having 1, 2, or 3 nucleotide differences therefrom, and the antisense strand comprises or consists of a sequence set forth in SEQ ID NO:74 or a nucleotide sequence having 1, 2, or 3 nucleotide differences therefrom;
the sense strand comprises or consists of a sequence set forth in SEQ ID NO:55 or a nucleotide sequence having 1, 2, or 3 nucleotide differences therefrom, and the antisense strand comprises or consists of a sequence set forth in SEQ ID NO:75 or a nucleotide sequence having 1, 2, or 3 nucleotide differences therefrom;
the sense strand comprises or consists of a sequence set forth in SEQ ID NO:56 or a nucleotide sequence having 1, 2, or 3 nucleotide differences therefrom, and the antisense strand comprises or consists of a sequence set forth in SEQ ID NO:76 or a nucleotide sequence having 1, 2, or 3 nucleotide differences therefrom;
the sense strand comprises or consists of a sequence set forth in SEQ ID NO:57 or a nucleotide sequence having 1, 2, or 3 nucleotide differences therefrom, and the antisense strand comprises or consists of a sequence set forth in SEQ ID NO:77 or a nucleotide sequence having 1, 2, or 3 nucleotide differences therefrom; or
the sense strand comprises or consists of a sequence set forth in SEQ ID NO:58 or a nucleotide sequence having 1, 2, or 3 nucleotide differences therefrom, and the antisense strand comprises or consists of a sequence set forth in SEQ ID NO:78 or a nucleotide sequence having 1, 2, or 3 nucleotide differences therefrom.

In preferred embodiments, the present invention provides an RNAi agent for inhibiting SCN10A gene expression, comprising a sense strand and an antisense strand that form a complementary duplex region, wherein the sense strand and antisense strand pair to form any one of the duplexes 51059, 51069, 51071, 51078, 51089, 51120, 51154, 51201, 51264, 51284, 51286, 51329, 51346, 51350, 51370, 51407, 51415, 51417, 51435, and 51441 as described herein.

### Nucleotide-Modified RNAi Agents

For any of the embodiments of the RNAi agents of the present invention described in the section "RNAi Agents For Inhibiting SCN10A Gene Expression", the sense strand and/or antisense strand of the RNAi agent may comprise at least one modified nucleotide.

In some embodiments, the sense strand and the antisense strand of the RNAi agent for inhibiting SCN10A gene expression provided by the present invention each comprise at least one modified nucleotide.

In some embodiments, each of the nucleotides in the sense strand and the antisense strand of the RNAi agent for inhibiting SCN10A gene expression provided by the present invention is modified.

In any of the above embodiments, the modified nucleotide is independently selected from the group consisting of: 2'-deoxy-thymidine (dT) nucleotide, 2'-O-methyl-modified nucleotide (2'-OMe), 2'-fluoro-modified nucleotide (2'-F), 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide (cEt), 2'-amino-modified nucleotide, 2'-O-allyl-modified nucleotide, 2'-C-alkyl-modified nucleotide, 2'-O-methoxyethyl-modified nucleotide (2'-MOE), abasic nucleotide, inverted abasic nucleotide, inverted nucleotide, morpholino nucleotide (MOP), phosphoramidate (PN), tetrahydropyran-modified nucleotide (THP), 1,5-anhydrohexitol-modified nucleotide (HNA), cyclohexenyl-modified nucleotide, nucleotide comprising a phosphorothioate group (PS), nucleotide comprising a methylphosphonate group, nucleotide comprising a 5'-phosphate, nucleotide comprising a 5'-phosphate mimic, cationic lipid-conjugated nucleotide, nucleotide comprising a 5'-vinylphosphonate (5'-VP), and combinations thereof.

In preferred embodiments, the modified nucleotides are independently selected from the group consisting of 2'-O-methyl-modified nucleotides, 2'-fluoro-modified nucleotides, nucleotides comprising phosphorothioate internucleoside linkages, and combinations thereof. In preferred embodiments, every nucleotide of the sense strand and/or the antisense strand of the RNAi agent is modified. In preferred embodiments, every nucleotide of the sense strand and/or the antisense strand of the RNAi agent is modified, and the modified nucleotides are independently selected from the group consisting of 2'-O-methyl-modified nucleotides, 2'-fluoro-modified nucleotides, nucleotides comprising phosphorothioate internucleoside linkages, and combinations thereof.

In preferred embodiments, each of the nucleotides in the sense strand and the antisense strand of the RNAi agent for inhibiting SCN10A gene expression provided by the present invention is modified, and the modification is selected from the group consisting of STC(Alnylam), ESC(Alnylam), Advanced ESC(Alnylam), ESC+(Alnylam), AD1-3(Arrowhead), AD5(Arrowhead) and GalXC(Dicerna) (see, e.g., Hu B, Zhong L, Weng Y, et al. Therapeutic siRNA: state of the art. Signal Transduct Target Ther. 2020; 5(1):101. Published 2020 Jun 19. doi:10.1038/s41392-020-0207-x).

In preferred embodiments, the 5'-terminal nucleotide of the antisense strand of the RNAi agent for inhibiting SCN10A gene expression provided by the present invention contains a phosphate or phosphate analog modification, preferably 5'-VP (see Parmar R, Willoughby JL, Liu J, et al. 5'-(E)-Vinylphosphonate: A Stable Phosphate Mimic Can Improve the RNAi Activity of siRNA-GalNAc Conjugates. Chembiochem. 2016;17(11):985-989).

In some embodiments, in the RNAi agent for inhibiting SCN10A gene expression provided by the present invention, each of the nucleotides of the antisense strand is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

In some embodiments, in the RNAi agent for inhibiting SCN10A gene expression provided by the present invention, each of the nucleotides of the antisense strand is modified, and in the 5' to 3' direction, the nucleotides at positions 2, 5, 7, 12, and 14 of the antisense strand are 2'-fluoro-modified nucleotides, while the nucleotides at remaining positions of the antisense strand are all 2'-methoxy-modified nucleotides.

In some embodiments, in the RNAi agent for inhibiting SCN10A gene expression provided by the present invention, each of the nucleotides of the antisense strand is modified, and in the 5' to 3' direction, the nucleotides at positions 2, 5, 7, 14, and 16 of the antisense strand are 2'-fluoro-modified nucleotides, while the nucleotides at remaining positions of the antisense strand are all 2'-methoxy-modified nucleotides.

In some embodiments, in the RNAi agent for inhibiting SCN10A gene expression provided by the present invention, each of the nucleotides of the antisense strand is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage. In preferable embodiments, the phosphorothioate internucleotide linkage is present at one or more of the following locations: (i) between nucleotide positions 1 and 2 at the 5'-end of the antisense strand; (ii) between nucleotide positions 2 and 3 at the 5'-end of the antisense strand; (iii) between nucleotide positions 1 and 2 at the 3'-end of the antisense strand; and (iv) between nucleotide positions 2 and 3 at the 3'-end of the antisense strand. In preferable embodiments, the phosphorothioate internucleotide linkage is present (i) between nucleotide positions 1 and 2 at the 5'-end of the antisense strand; (ii) between nucleotide positions 2 and 3 at the 5'-end of the antisense strand; (iii) between nucleotide positions 1 and 2 at the 3'-end of the antisense strand; and (iv) between nucleotide positions 2 and 3 at the 3'-end of the antisense strand.

In some embodiments, in the RNAi agent for inhibiting SCN10A gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides.

For example, in the RNAi agent for inhibiting SCN10A gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 5, 7, 8, and 9 of the sense strand are 2'-fluoro-modified nucleotides, while the nucleotides at remaining positions of the sense strand are all 2'-methoxy-modified nucleotides.

For example, in the RNAi agent for inhibiting SCN10A gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7, 8, and 9 of the sense strand are 2'-fluoro-modified nucleotides, while the nucleotides at remaining positions of the sense strand are all 2'-methoxy-modified nucleotides.

For example, in the RNAi agent for inhibiting SCN10A gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 5, 7, and 9 of the sense strand are 2'-fluoro-modified nucleotides, while the nucleotides at remaining positions of the sense strand are all 2'-methoxy-modified nucleotides.

For example, in the RNAi agent for inhibiting SCN10A gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides; and in the 5' to 3' direction, the nucleotides at positions 7, 9, and 11 of the sense strand are 2'-fluoro-modified nucleotides, while the nucleotides at remaining positions of the sense strand are all 2'-methoxy-modified nucleotides.

In preferred embodiments, the sense strand has at least one phosphorothioate internucleoside linkage. In preferred embodiments, the phosphorothioate internucleoside linkage is present in one or more of the following positions: (i) between nucleotide position 1 and nucleotide position 2 at the 5' end of the sense strand; (ii) between nucleotide position 2 and nucleotide position 3 at the 5' end of the sense strand; (iii) between nucleotide position 1 and nucleotide position 2 at the 3' end of the sense strand; and (iv) between nucleotide position 2 and nucleotide position 3 at the 3' end of the sense strand. In more preferred embodiments, the phosphorothioate internucleoside linkages are present between nucleotide position 1 and nucleotide position 2 at the 5' end of the sense strand; and between nucleotide position 2 and nucleotide position 3 at the 5' end of the sense strand. In more preferred embodiments, the phosphorothioate internucleoside linkages are present between nucleotide position 1 and nucleotide position 2 at the 5' end of the sense strand; between nucleotide position 2 and nucleotide position 3 at the 5' end of the sense strand; between nucleotide position 1 and nucleotide position 2 at the 3' end of the sense strand; and between nucleotide position 2 and nucleotide position 3 at the 3' end of the sense strand.

In preferred embodiments, the antisense strand has at least one phosphorothioate internucleoside linkage. Preferably, the phosphorothioate internucleoside linkage is present in one or more of the following: between nucleotide position 1 and nucleotide position 2 at the 5' end of the antisense strand; between nucleotide position 2 and nucleotide position 3 at the 5' end of the antisense strand; between nucleotide position 1 and nucleotide position 2 at the 3' end of the antisense strand; and between nucleotide position 2 and nucleotide position 3 at the 3' end of the antisense strand.

Accordingly, in some embodiments, in the RNAi agent for inhibiting SCN10A gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage, wherein the phosphorothioate internucleotide linkage is present at one or more of the following locations: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides; and the sense strand comprises at least one phosphorothioate internucleotide linkage, wherein the phosphorothioate internucleotide linkage is present between nucleotide positions 1 and 2 at the 5'-end of the sense strand and/or between nucleotide positions 2 and 3 at the 5'-end of the sense strand.

In some embodiments, in the RNAi agent for inhibiting SCN10A gene expression provided by the present invention, each of the nucleotides of the sense and antisense strands is modified, and in the 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides, and the antisense strand comprises at least one phosphorothioate internucleotide linkage, wherein the phosphorothioate internucleotide linkage is present: between nucleotide positions 1 and 2 at the 5'-end of the antisense strand; between nucleotide positions 2 and 3 at the 5'-end of the antisense strand; between nucleotide positions 1 and 2 at the 3'-end of the antisense strand; and between nucleotide positions 2 and 3 at the 3'-end of the antisense strand; and in the 5' to 3' direction, the nucleotides at positions 7 and 9 of the sense strand are 2'-fluoro-modified nucleotides, and one or two of the nucleotides at positions 5, 8, and 11 of the sense strand is (are) 2'-fluoro-modified nucleotide(s), while the nucleotides at remaining positions of the sense strand are all 2'-methoxy-modified nucleotides; and the sense strand comprises at least one phosphorothioate internucleotide linkage, wherein the phosphorothioate internucleotide linkage is present between nucleotide positions 1 and 2 at the 5'-end of the sense strand and between nucleotide positions 2 and 3 at the 5'-end of the sense strand.

### Ligand-Conjugated RNAi Agents

For any of the embodiments of the RNAi agent of the present invention described in the preceding sections entitled "RNAi Agents For Inhibiting SCN10A Gene Expression" and "Nucleotide-modified RNAi agents", the RNAi agent may comprise a ligand. As used herein, a "ligand" refers to any compound or molecule capable of interacting, directly or indirectly, with another compound or molecule. The interaction of a ligand with another compound or molecule may elicit a biological response (e.g., initiation of a signal transduction cascade, induction of receptor-mediated endocytosis) or may simply be a physical attachment. A ligand may alter one or more properties of the attached double-stranded RNA molecule, such as the pharmacodynamics, pharmacokinetics, binding, absorption, cellular distribution, cellular uptake, charge, and/or clearance of the RNA molecule.

The SCN10A gene is expressed in the various cells and tissues. Therefore, in certain embodiments, it is desirable to specifically deliver the ASO or RNAi agents of the present invention to hepatocytes. Accordingly, in certain embodiments, the ligand targets the specific delivery of the ASO or RNAi agent to hepatocytes using various methods described in more detail below. In certain embodiments, the ASO or RNAi agent is targeted to hepatocytes with a ligand that binds the surface-expressed asialoglycoprotein receptor (ASGR) or a component thereof (e.g., ASGR1, ASGR2).

In some embodiments, the ASO or RNAi agent can be specifically targeted to the liver by using a ligand that binds or interacts with a protein expressed on the surface of hepatocytes. For example, in certain embodiments, the ligand can comprise an antigen-binding protein (e.g., an antibody or binding fragment thereof such as Fab, scFv) that specifically binds a receptor expressed on hepatocytes, such as the asialoglycoprotein receptor and the LDL receptor. In one particular embodiment, the ligand comprises an antibody or binding fragment thereof that specifically binds ASGR1 and/or ASGR2. In another embodiment, the ligand comprises a Fab fragment of an antibody that specifically binds ASGR1 and/or ASGR2. In another embodiment, the ligand comprises a single-chain variable antibody fragment (scFv fragment) of an antibody that specifically binds ASGR1 and/or ASGR2. Exemplary antibodies that specifically bind ASGR1 and binding fragments thereof, which can serve as ligands for targeting the RNAi agents of the present invention to the liver, are described in WO 2017/058944, which is incorporated herein by reference in its entirety. Other antibodies or binding fragments thereof that specifically bind ASGR1, the LDL receptor, or other liver surface-expressed proteins suitable for use as ligands in the RNAi agents of the present invention are available from commercial sources.

In some embodiments, when used for extrahepatic delivery, including but not limited to, the central nervous system (CNS) (e.g., brain, spinal cord, or eye), muscle, lung, or adipose tissue, the RNAi agent may comprise one or more lipophilic groups conjugated to one or more positions of the antisense strand and/or sense strand. For example, the lipophilic group may be linked to the antisense strand and/or sense strand via a nucleobase, sugar moiety, or internucleoside linkage. In some embodiments, a lipophilic group having a phosphoramidite group is coupled to the 3' end or the 5' end of the sense strand or antisense strand in the final synthesis cycle. In some embodiments, the lipophilic group has an octanol-water partition coefficient exceeding 0, 1, 1.5, 2, 3, 4, 5, or 10. In some embodiments, the ligand is a lipophilic group, which is preferably selected from: lipids, vitamins, steroids, C₅-C₃₀ saturated or unsaturated fatty acids, C₅-C₃₀ alkyl groups, and polypeptides comprising at least one positively charged amino acid residue; said lipophilic group is more preferably selected from cholesterol, C₁₆ saturated or unsaturated fatty acids, C₁₆ alkyl groups, C₂₂ saturated or unsaturated fatty acids, or C₂₂ alkyl groups. Suitable lipophilic groups may be aliphatic, alicyclic, polyalicyclic compounds, steroids, linear or branched aliphatic hydrocarbon groups.

Exemplary lipophilic groups include, for example, lipophilic groups Y132 to Y135, Y158, Y165 to Y168, L10, L57, L321, L322, Q361 to Q367, Q361s to Q367s, Q370, Q377 to Q379, Q383, etc., as described in WO 2021/092371. Other ligands that may be conjugated to the antisense oligonucleotide or RNAi agent of the present invention can be found in WO 2017/053995, WO 2019/217459, WO 2021/092371, WO 2017/053995, WO 2010/039548, WO 2023/064530, WO 2022/213118, and WO 2019/079386, each of which is incorporated herein by reference in its entirety.

In certain embodiments, the ligand comprises a carbohydrate. A "carbohydrate" refers to a compound composed of one or more monosaccharide units having at least 6 carbon atoms (which can be linear, branched, or cyclic) with oxygen, nitrogen, or sulfur atoms attached to each carbon atom. Carbohydrates include, but are not limited to, sugars (e.g., monosaccharides, disaccharides, trisaccharides, tetrasaccharides, and oligosaccharides containing about 4, 5, 6, 7, 8, or 9 monosaccharide units) and polysaccharides (such as starch, glycogen, cellulose, and polysaccharide gums). In some embodiments, the carbohydrate incorporated into the ligand is selected from pentoses, hexoses, or heptoses, and disaccharides and trisaccharides including such monosaccharide units. In other embodiments, the carbohydrate incorporated into the ligand is an amino sugar, such as galactosamine, glucosamine, N-acetylgalactosamine, and N-acetylglucosamine.

In some embodiments, the ligand comprises a hexose or a hexosamine. The hexose can be selected from glucose, galactose, mannose, fucose, or fructose. The hexosamine can be selected from fructosamine, galactosamine, glucosamine, or mannosamine. In certain embodiments, the ligand comprises glucose, galactose, galactosamine, or glucosamine. In one embodiment, the ligand comprises glucose, glucosamine, or N-acetylglucosamine. In another embodiment, the ligand comprises galactose, galactosamine, or N-acetylgalactosamine. In particular embodiments, ligands comprising glucose, galactose, and N-acetylgalactosamine (GalNAc) are particularly effective in targeting RNA to hepatocytes, as these ligands bind the ASGR expressed on the surface of hepatocytes. Examples of GalNAc- or galactose-containing ligands that can be incorporated into the RNAi agents of the present invention are described in U.S. Pat. Nos. 7,491,805, 8,106,022, and 8,877,917; U.S. Patent Application Publication No. US 20030130186; and WIPO Publication No. WO 2013/166155, all of which are incorporated herein by reference in their entirety.

In certain embodiments, the ligand comprises a multivalent carbohydrate moiety. As used herein, a "multivalent carbohydrate moiety" refers to a moiety comprising two or more carbohydrate units capable of independently binding or interacting with other molecules. For example, a multivalent carbohydrate moiety includes two or more binding domains composed of carbohydrates that can bind to two or more different molecules or two or more different sites on the same molecule. The "valency" of a carbohydrate moiety indicates the number of individual binding domains within the carbohydrate moiety. For example, the terms "monovalent," "bivalent," "trivalent," and "tetravalent" relative to a carbohydrate moiety refer to carbohydrate moieties having one, two, three, and four binding domains, respectively. A multivalent carbohydrate moiety can comprise a multivalent lactose moiety, a multivalent galactose moiety, a multivalent glucose moiety, a multivalent N-acetylgalactosamine moiety, a multivalent N-acetylglucosamine moiety, a multivalent mannose moiety, or a multivalent fucose moiety. In some embodiments, the ligand comprises a multivalent galactose moiety. In other embodiments, the ligand comprises a multivalent N-acetylgalactosamine moiety. In these and other embodiments, the multivalent carbohydrate moiety can be bivalent, trivalent, or tetravalent. In such embodiments, the multivalent carbohydrate moiety can be bi-branched or tri-branched. In one particular embodiment, the multivalent N-acetylgalactosamine moiety is trivalent or tetravalent. In another particular embodiment, the multivalent galactose moiety is trivalent or tetravalent. Exemplary trivalent and tetravalent GalNAc-containing ligands for incorporation into the ASO or RNAi agents of the present invention are described in detail below.

The ligand may also comprise an integrin ligand, for example, a ligand that specifically binds to an integrin, including but not limited to αVβ6. Suitable integrin ligands include, for example, those integrin ligands mentioned in WO2019089765A1 or WO2022056286A1, which are incorporated herein by reference in their entirety.

The ligand may also comprise a transferrin receptor 1 (TfR1) ligand, such as an anti-TfR1 antibody or polypeptide. Suitable TfR1 ligands include, for example, TfR1 antibodies described in WO2022147209A1 or WO2021154476A1, or bicyclic peptide ligands described in WO2022101633A1, and these patent documents are incorporated herein by reference in their entirety.

The ligand can be attached or conjugated to the RNA molecule of the ASO or RNAi agent directly or indirectly. For example, in some embodiments, the ligand is directly covalently attached to the ASO ro the sense strand or the antisense strand of the RNAi agent. In other embodiments, the ligand is covalently attached to the ASO or the sense strand or the antisense strand of the RNAi agent via a linker. The ligand can be attached to a nucleobase, sugar moiety, or internucleotide linkage of ASO or the sense strand or antisense strand of the RNAi agent of the present invention.

In some embodiments, the ligand can be attached to 3' or 5'-end of the sense strand or antisense strand. In certain embodiments, the ligand is covalently attached to 5'-end of the sense strand. In such embodiments, the ligand is attached to 5'-terminal nucleotide of the sense strand. In these and other embodiments, the ligand is attached at 5'-position of 5'-terminal nucleotide of the sense strand. In other embodiments, the ligand is covalently attached to 3'-end of the sense strand. For example, in some embodiments, the ligand is attached to 3'-terminal nucleotide of the sense strand. In certain such embodiments, the ligand is attached at 3'-position of 3'-terminal nucleotide of the sense strand. In alternative embodiments, the ligand is attached near 3'-end but prior to one or more terminal nucleotides (i.e., one, two, three, or four nucleotides from the terminus). In some embodiments, the ligand is attached at the 2'-position of the sugar of 3'-terminal nucleotide of the sense strand. In other embodiments, the ligand is attached at the 2'-position of the sugar of 5'-terminal nucleotide of the sense strand.

In certain embodiments, the ligand is attached to the sense strand or antisense strand via a linker. A "linker" refers to an atom or group of atoms that covalently attaches the ligand to the polynucleotide component of the ASO or RNAi agent. The linker can have a length of about 1 to about 30 atoms, about 2 to about 28 atoms, about 3 to about 26 atoms, about 4 to about 24 atoms, about 6 to about 20 atoms, about 7 to about 20 atoms, about 8 to about 20 atoms, about 8 to about 18 atoms, and about 12 to about 18 atoms. In some embodiments, the linker can comprise a bifunctional linking moiety, typically comprising an alkyl group with two functional groups. One functional group is chosen to bind to the compound of interest (e.g., the sense strand or antisense strand of the RNAi agent), while the other functional group is chosen to bind to essentially any selected group, such as the ligands described herein. In certain embodiments, the linker comprises an oligomer of a chain structure or repeating units, such as ethylene glycol or amino acid units. Examples of functional groups commonly used in bifunctional linking moieties include, but are not limited to, electrophiles for reaction with nucleophilic groups and nucleophiles for reaction with electrophilic groups. In some embodiments, the bifunctional linking moiety includes an amino group, a hydroxyl group, a carboxylic acid, a thiol, an unsaturated bond (e.g., a double or triple bond), and the like.

Linkers suitable for attaching a ligand to the ASO or sense strand or antisense strand of the RNAi agent of the present invention include, but are not limited to, pyrrolidine, 8-amino-3,6-dioxaoctanoic acid, succinimidyl-4-(N-maleimidomethyl)cyclohexane-1-carboxylate, 6-aminohexanoic acid, substituted C₁-C₂₀ alkyl, substituted or unsubstituted C₂-C₂₀ alkenyl, or substituted or unsubstituted C₂-C₂₀ alkynyl. Preferred substituents for such linkers include, but are not limited to, hydroxyl, amino, alkoxy, carboxyl, benzyl, phenyl, nitro, thiol, thioalkoxy, halogen, alkyl, aryl, alkenyl, and alkynyl.

In certain embodiments, the linker is cleavable. A cleavable linker is one that is sufficiently stable outside the cell but is cleaved to release the two parts linked together by the linker upon entry into a target cell. In some embodiments, the cleavable linker is cleaved at least 10, 20, 30, 40, 50, 60, 70, 80, 90 times or more, or at least 100 times faster in the target cell or under a first reference condition (which can, for example, be selected to mimic or represent intracellular conditions) than in the blood of a subject.

Cleavable linkers are susceptible to cleavage agents, such as pH, redox potential, or the presence of degradative molecules. Generally, cleavage agents are more prevalent or found at higher levels or activity inside cells than in serum or blood. Examples of such cleavage agents include: redox agents, selected for specific substrates or lacking substrate specificity, including, for example, oxidases or reductases present in cells; esterases; endosomes or agents that can create an acidic environment, such as those resulting in a pH of 5 or lower; enzymes that can hydrolyze or degrade acid-cleavable linkers acting as general acids, peptidases (which can be substrate-specific), and phosphatases.

A cleavable linker can comprise a pH-sensitive moiety. Human serum has a pH of 7.4, while the average intracellular pH is slightly lower, ranging from about 7.1-7.3. Endosomes have a more acidic pH, in the range of 5.5-6.0, and lysosomes have an even more acidic pH (around 5.0). Some linkers will have a cleavable group that is cleaved at a preferred pH, thereby releasing the RNA molecule from the ligand inside the cell, or into a desired cellular organelle. The linker can include a cleavable group that is cleaved by a specific enzyme. The type of cleavable group incorporated into the linker can depend on the cell to be targeted. For example, a liver-targeting ligand can be linked to the RNA molecule via a linker comprising of an ester group. Hepatocytes are rich in esterases, so the linker is cleaved more efficiently in hepatocytes than in cell types not rich in esterases. Other cell types rich in esterases include cells of the lung, renal cortex, and testis. When targeting cells rich in peptidases, such as hepatocytes and synoviocytes, linkers containing peptide bonds can be used.

Other types of linkers suitable for attaching ligands to the sense strand or antisense strand in the RNAi agents of the present invention are known in the art, such as those described in U.S. Pat. Nos. 7,723,509, 8,017,762, 8,828,956, 8,877,917, and 9,181,551, all of which are incorporated herein by reference in their entirety.

In certain embodiments, the ligand covalently attached to the ASO or the sense strand or antisense strand of the RNAi agent of the present invention comprises a GalNAc moiety, such as a multivalent GalNAc. In some embodiments, the multivalent GalNAc moiety is a trivalent GalNAc and is attached to 3'-end of the sense strand. In other embodiments, the multivalent GalNAc moiety is a trivalent GalNAc and is attached to 5'-end of the sense strand. In other embodiments, the multivalent GalNAc moiety is a tetravalent GalNAc moiety and is attached to 3'-end of the sense strand. In other embodiments, the multivalent GalNAc moiety is a tetravalent GalNAc moiety and is attached to 5'-end of the sense strand.

In some embodiments, the ligand is L96, NAG25, and NAG37, having the formulas shown below, respectively, wherein the wave line represents a site to which the ASO or the sense strand or antisense strand of the RNAi agent is attached.

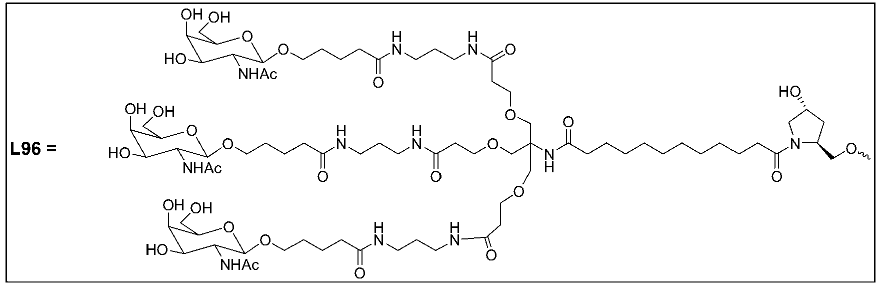

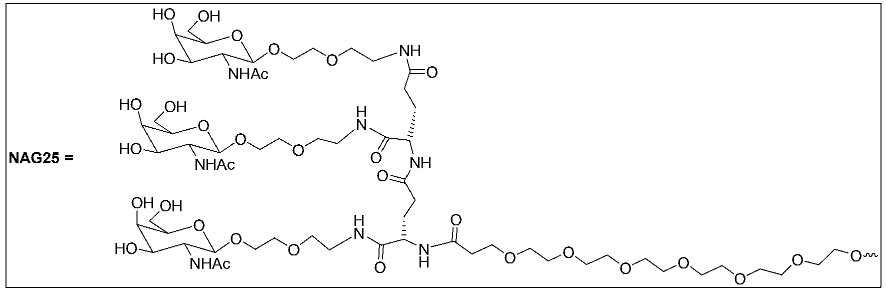

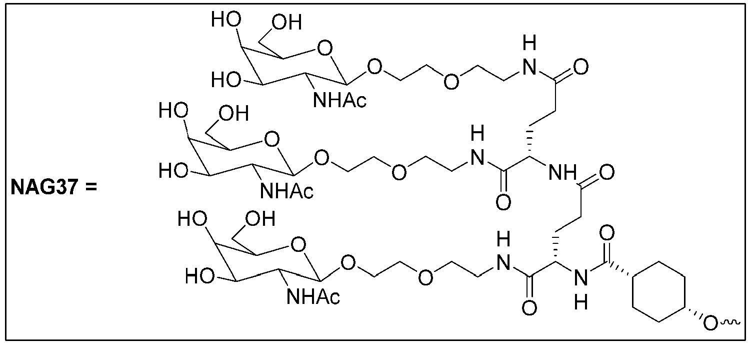

### Pharmaceutical Compositions

The present invention also encompasses pharmaceutical compositions and formulations comprising the antisense oligonucleotides or RNAi agents described herein and a pharmaceutically acceptable carrier, excipient, or diluent. Such compositions and formulations can be used to reduce the expression of the SCN10A gene in a patient in need thereof. Considering clinical application, the pharmaceutical compositions and formulations will be prepared in a form suitable for the intended application. Typically, this will involve preparing compositions substantially free of pyrogens and other impurities that may be harmful to humans or animals.

The ingredients and methods for formulating pharmaceutical compositions depend on many criteria, including, but not limited to, the route of administration, the type and extent of the disease or condition to be treated, or the dosage to be administered. In some embodiments, the pharmaceutical composition is formulated based on the intended delivery route. For example, in certain embodiments, the pharmaceutical composition is formulated for parenteral delivery. Parenteral administration forms include intravenous, intra-arterial, subcutaneous, intrathecal, intraperitoneal, or intramuscular injection or infusion. In one embodiment, the pharmaceutical composition is formulated for intravenous delivery. In such embodiments, the pharmaceutical composition may comprise a lipid-based delivery vehicle. In another embodiment, the pharmaceutical composition is formulated for subcutaneous delivery. In such embodiments, the pharmaceutical composition may comprise a targeting ligand (e.g., a GalNAc-containing or antibody-containing ligand as described herein).

In some embodiments, the pharmaceutical composition comprises an effective amount of the antisense oligonucleotides or RNAi agent described herein. An "effective amount" refers to an amount sufficient to produce a beneficial or desired clinical outcome. In some embodiments, the effective amount is an amount sufficient to reduce the expression of the SCN10A gene in a specific tissue or cell type of the patient (e.g., the liver or hepatocytes). The effective amount of the antisense oligonucleotides or RNAi agent of the present invention can be from about 0.01 mg/kg body weight to about 100 mg/kg body weight, and can be administered daily, weekly, monthly, or at longer intervals. The precise determination of the specific effective dosage amount and administration frequency may be based on several factors, including the patient's body size, age, and general condition, the type of disease to be treated (e.g., myocardial infarction, coronary artery disease, peripheral artery disease, stroke), the specific antisense oligonucleotides or RNAi agent used, and the route of administration.

Administration of the pharmaceutical compositions of the invention can be by any common route provided the target tissue is accessible via that route. These routes include, but are not limited to, parenteral (e.g., subcutaneous, intramuscular, intraperitoneal, or intravenous), oral, nasal, buccal, intradermal, transdermal, and sublingual routes, or by direct injection into liver tissue or delivery via the hepatic portal vein. In some embodiments, the pharmaceutical composition is administered parenterally. For example, in certain embodiments, the pharmaceutical composition is administered intravenously. In other embodiments, the pharmaceutical composition is administered subcutaneously.

Colloidal dispersion systems can be used as delivery vehicles for the antisense oligonucleotides or RNAi agents of the present invention, such as macromolecular complexes, nanocapsules, microspheres, beads, and lipid-based systems, including oil-in-water emulsions, micelles, mixed micelles, and liposomes. Commercially available fat emulsions suitable for delivering the nucleic acids of the present invention include Intralipid (Baxter International Inc.), Liposyn (Abbott Pharmaceuticals), Lipsyn II (Hospira), Liposyn III (Hospira), Nutrilipid (B. Braun Medical Inc.), and other similar fat emulsions. A preferred colloidal system for use as an *in vivo* delivery vehicle is the liposome (i.e., an artificial membrane vesicle). The RNAi agents of the present invention can be encapsulated within liposomes or can form complexes therewith, particularly with cationic liposomes. Alternatively, the RNAi agents of the present invention can be complexed with lipids, particularly with cationic lipids. Suitable cationic lipids are, for example, dioleoyltrimethylammonium propane (DOTAP) and dioleoylphosphatidylethanolamine (DOTMA).

Liposomal formulations are particularly suitable for topical administration, with liposomes conferring several advantages over alternative formulations. Such advantages include reduced incidence of adverse effects associated with high systemic absorption of administered therapeutics, enhanced accumulation of administered therapeutics at desired target sites, and capability for dermal delivery of antisense oligonucleotides or RNAi agents. In some embodiments, liposomes are employed to deliver RNAi agents to epidermal cells while concurrently enhancing permeation of said antisense oligonucleotides or RNAi agents into dermal tissues, e.g., within the skin.

The antisense oligonucleotides or RNAi agents of the present invention may be fully encapsulated within lipid-based formulations, such as LNPs or other nucleic acid-lipid particles. As used herein, the term "LNP" refers to stable nucleic acid-lipid particles. LNPs typically comprise a cationic lipid, a non-cationic lipid, and a lipid that prevents particle aggregation (e.g., a PEG-lipid conjugate). LNPs are particularly useful for systemic applications as they exhibit prolonged circulation times following intravenous (i.v.) injection and accumulate at distal sites (e.g., sites physically separated from the administration site). LNPs include "pSPLP," which comprise encapsulated condensing agent-nucleic acid complexes as described in WO00/03683. The LNP particles of the present invention generally possess a mean diameter ranging from about 50 nm to about 150 nm, more typically from about 60 nm to about 130 nm, more typically from about 70 nm to about 110 nm, and most typically from about 70 nm to about 90 nm, and are substantially non-toxic. Additionally, when nucleic acids are present within the nucleic acid-lipid particles of the invention, they demonstrate resistance to nuclease degradation in aqueous solutions. Nucleic acid-lipid particles and methods for their preparation are disclosed in, for example, U.S. Pat. Nos. 5,976,567; 5,981,501; 6,534,484; 6,586,410; 6,815,432; U.S. Publication No. 2010/0324120; and WO 96/40964. In one embodiment, the lipid-to-drug ratio (mass/mass basis) (e.g., lipid-to-RNAi agent ratio) will range from about 1:1 to about 50:1, about 1:1 to about 25:1, about 3:1 to about 15:1, about 4:1 to about 10:1, about 5:1 to about 9:1, or about 6:1 to about 9:1.

Cationic lipids may include, for example, N,N-dioleyl-N,N-dimethylammonium chloride (DODAC), N,N-distearyl-N,N-dimethylammonium bromide (DDAB), N-(1-(2,3-dioleyloxy)propyl)-N,N,N-trimethylammonium chloride (DOTAP), N-(1-(2,3-dioleyloxy)propyl)-N,N,N-trimethylammonium chloride (DOTMA), N,N-dimethyl-2,3-dioleyloxy)propylamine (DODMA), 1,2-dioleyloxy-N,N-dimethylaminopropane (DLinDMA), 1,2-dilinoleyloxy-N,N-dimethylaminopropane (DLenDMA), 1,2-dilinoleylcarbamoyloxy-3-dimethylaminopropane (DLin-C-DAP), 1,2-dilinoleyloxy-3-(dimethylamino)acetoxypropane (DLin-DAC), 1,2-dilinoleyloxy-3-morpholinopropane (DLin-MA), 1,2-dilinolenyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleoyloxy-3-(N-methylpiperazinyl)propane (DLin-MPZ), 3-(N,N-dilinoleylamino)-1,2-propanediol (DLinAP), 3-(N,N-dioleylamino)-1,2-propanediol (DOAP), 1,2-dilinoleyloxo-3-(2-N,N-dimethylamino)ethoxypropane (DLin-EG-DMA), 1,2-dilinolenyloxy-N,N-dimethylaminopropane (DLinDMA), 2,2-dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-(bis(2-hydroxydodecyl)amino)ethyl)(2-hydroxydodecyl)amino)ethyl)piperazin-1-yl)ethylazanediyl)didodecan-2-ol (Tech G1), or mixtures thereof. The cationic lipid may constitute about 20 mol% to about 50 mol%, or about 40 mol% of the total lipid present in the particle.

In some embodiments, the compound 2,2-dilinoleyl-4-dimethylaminomethyl-[1,3]-dioxolane may be utilized in the preparation of lipid-siRNA nanoparticles. In certain embodiments, the lipid-siRNA particles comprise 40% 2,2-dilinoleyl-4-dimethylaminomethyl-[1,3]-dioxolane : 10% DSPC : 40% cholesterol : 10% PEG-C-DOMG (mol%), with a particle size of 63.0 ± 20 nm and an siRNA-to-lipid ratio of 0.027.

Ionizable/non-cationic lipids may be anionic lipids or neutral lipids, including but not limited to distearoylphosphatidylcholine (DSPC), dioleoylphosphatidylcholine (DOPC), dipalmitoylphosphatidylcholine (DPPC), dioleoylphosphatidylglycerol (DOPG), dipalmitoyl (DP), dioleoylphosphatidylethanolamine (DOPE), palmitoyloleoylphosphatidylcholine (POPC), palmitoyloleoylphosphatidylethanolamine (POPE), N-(4-(p-maleimidophenyl)butyryl)-dioleoylphosphatidylethanolamine (DOPE-mal), dipalmitoylphosphatidylethanolamine (DPPE), dimyristoylphosphatidylethanolamine (DMPE), distearoylphosphatidylethanolamine (DSPE), 16-O-monomethyl PE, 16-O-dimethyl PE, 18-1-trans PE, 1-stearoyl-2-oleoyl-phosphatidylethanolamine (SOPE), cholesterol, or mixtures thereof. The non-cationic lipid may constitute about 5 mol% to about 90 mol%, about 10 mol%, or about 58 mol% (when cholesterol is included) of the total lipid present in the particle.

Lipids conjugated to prevent particle aggregation may include, for example, polyethylene glycol (PEG)-lipids, comprising but not limited to PEG-diacylglycerol (PEG-DAG), PEG-dialkoxypropyl (PEG-DAA), PEG-phospholipid, PEG-ceramide (PEG-Cer), or mixtures thereof. PEG-DAA conjugates may include, for instance, PEG-didodecyloxypropyl (C12), PEG-dimyristyloxypropyl (C14), PEG-dipalmityloxypropyl (C14), or PEG-distearoyloxypropyl (C18). The lipid conjugated to prevent particle aggregation may constitute 0 mol% to about 20 mol%, or about 2 mol% of the total lipid present in the particle. In some embodiments, the nucleic acid-lipid particles further comprise cholesterol, for example constituting about 10 mol% to about 60 mol%, or about 48 mol% of the total lipid present in said particle.

In one embodiment, the lipidoid ND98·4HCl (molecular weight 1487) (see U.S. Patent Application No. 12/056,230, incorporated herein by reference in its entirety), cholesterol (Sigma-Aldrich), and PEG-ceramide C16 (Avanti Polar Lipids) may be utilized to prepare lipid-dsRNA nanoparticles (i.e., LNP01 particles). Each stock solution in ethanol may be prepared as follows: ND98 at 133 mg/mL; cholesterol at 25 mg/mL; PEG-ceramide C16 at 100 mg/mL. The stock solutions of ND98, cholesterol, and PEG-ceramide C16 may then be mixed at a mol ratio of, for example, 42:48:10. The combined lipid solution may be mixed with aqueous siRNA (e.g., in sodium acetate buffer at pH 5) such that the final ethanol concentration is about 35-45% and the final sodium acetate concentration is about 100-300 mM. Lipid-siRNA nanoparticles typically form spontaneously upon mixing.

Other exemplary lipid-siRNA formulations are available from *e.g.* WO2009/127060(SNALP), PCT/US2010/022614(XTC), US 2010/0324120(MC3), PCT/US09/63933(ALNY-100) and WO2010/129709(C12-200).

In some embodiments, the antisense oligonucleotide or RNAi agent of the present invention may be conjugated to a cell-penetrating peptide (CPP), wherein said CPP may comprise at least one positively charged amino acid or ionizable amino acid, such as arginine, to form a peptide-oligonucleotide conjugate. Suitable CPPs for forming peptide-oligonucleotide conjugates may be found, for example, in those described in WO2022213118A1 or WO2019079386A1, which are incorporated herein by reference in their entirety.

Pharmaceutical compositions suitable for injectable use comprise, for example, sterile aqueous solutions or dispersions and sterile powders for the extemporaneous preparation of sterile injectable solutions or dispersions. Generally, such formulations are sterile and possess sufficient fluidity for ease of syringeability. The formulations shall remain stable under manufacturing and storage conditions and shall be preserved against the contaminating action of microorganisms such as bacteria and fungi. Suitable solvents or dispersion media may include, for instance, water, ethanol, polyhydric alcohols (e.g., glycerol, propylene glycol, liquid polyethylene glycols, and the like), suitable mixtures thereof, and vegetable oils. Appropriate fluidity may be maintained, for example, using a coating material such as lecithin, by maintaining required particle size in dispersions, or by employing surfactants. Prevention of microbial action may be achieved through various antibacterial and antifungal agents, including but not limited to parabens, chlorobutanol, phenol, sorbic acid, thimerosal, and the like. In many cases, it is preferable to include isotonic agents, such as sugars or sodium chloride. Prolonged absorption of injectable compositions may be accomplished by incorporating agents delaying absorption in the composition, for example, aluminum monostearate and gelatin.

Sterile injectable solutions may be prepared by incorporating the requisite quantity of the active compound with any additional ingredients (e.g., those enumerated supra) into an appropriate solvent, followed by filtration sterilization. Generally, dispersions are prepared by incorporating various sterilized active ingredients into a sterile vehicle medium containing essential dispersion agents and other requisite components, for instance, as described above. In the case of sterile powders for preparing sterile injectable solutions, preferred preparation methods include vacuum desiccation and lyophilization techniques, which yield powders of the active ingredient(s) together with any additional desired constituents from their prior sterile-filtered solutions.

The compositions of the present invention may generally be formulated in neutral form or as pharmaceutically acceptable salts. Such salts include, for example, acid addition salts (formed with free amino groups) derived from inorganic acids (e.g., hydrochloric or phosphoric acids) or organic acids (e.g., acetic, oxalic, tartaric, mandelic acids, etc.). Salts formed with free carboxyl groups may likewise be derived from inorganic bases (e.g., sodium, potassium, ammonium, calcium, or ferric hydroxides) or organic bases (e.g., isopropylamine, trimethylamine, histidine, procaine, etc.). In some embodiments, the antisense oligonucleotide or RNAi agent of the invention is formulated as a sodium salt.

For parenteral administration in aqueous solution form, for example, the solution is typically appropriately buffered, and the liquid diluent is first rendered isotonic with sufficient saline or glucose, for instance. Such aqueous solutions may be employed for intravenous, intramuscular, subcutaneous, and intraperitoneal administration, among others. Preferably, sterile aqueous media are utilized. By way of example, a unit dose may be dissolved in 1 mL of isotonic NaCl solution and either added to 1000 mL of subcutaneous infusion fluid or injected at the proposed infusion site. For administration to humans, the formulations shall comply with sterility, pyrogenicity, general safety, and purity standards as required by local Food and Drug Administration regulations. In certain embodiments, the pharmaceutical composition of the invention comprises the sterile saline solution described herein and the RNAi agent, or consists essentially of these components. In other embodiments, the pharmaceutical composition of the invention comprises the antisense oligonucleotide or RNAi agent described herein and sterile water (e.g., Water for Injection, WFI), or consists essentially thereof. In yet other embodiments, the pharmaceutical composition of the invention comprises the antisense oligonucleotide or RNAi agent described herein and Phosphate-Buffered Saline (PBS), or consists essentially thereof.

In certain embodiments, the pharmaceutical compositions of the present invention are packaged with or stored within an administration device. Devices for injectable formulations comprise, but are not limited to, injection ports, pre-filled syringes, auto-injectors, infusion pumps, implantable injectors, and injection pens. Devices for nebulized or powdered formulations comprise, but are not limited to, inhalers, insufflators, nebulizers, and the like. Accordingly, the present invention encompasses an administration device containing the pharmaceutical composition of the invention, for treating or preventing one or more diseases or disorders as described herein.

### Therapeutic Methods and Uses

The present invention provides a method for reducing or inhibiting the expression of SCN10A gene in a cell (e.g., a hepatocyte) by contacting the cell with any of the antisense oligonucleotides or RNAi agents described herein. The cell can be *in vitro* or *in vivo*. SCN10A gene expression can be assessed by measuring the amount or level of SCN10A mRNA or SCN10A protein. The reduction in SCN10A expression in cells or animals treated with the antisense oligonucleotides or RNAi agents of the invention can be determined relative to SCN10A expression in cells and animals not treated with the antisense oligonucleotide or RNAi agent or treated with a control antisense oligonucleotide or RNAi agent. For example, in some embodiments, the reduction in SCN10A expression is assessed by (a) measuring the amount or level of SCN10A mRNA in hepatocytes treated with an antisense oligonucleotide or RNAi agent of the invention, (b) measuring the amount or level of SCN10A mRNA in hepatocytes treated with a control RNAi agent (e.g., an RNAi agent targeting an RNA molecule not expressed in hepatocytes or an antisense oligonucleotide or RNAi agent having a nonsense or scrambled sequence) or no antisense oligonucleotide or RNAi agent, and (c) comparing the SCN10A mRNA level measured in the treated cells of (a) with the SCN10A mRNA level in the control cells of (b). The SCN10A mRNA levels in the treated and control cells may be normalized to the RNA level of a control gene (e.g., 18S ribosomal RNA or a housekeeping gene) prior to comparison. SCN10A mRNA levels can be measured by various methods, including Northern blot analysis, nuclease protection assay, fluorescent in situ hybridization (FISH), reverse transcriptase (RT)-PCR, real-time RT-PCR, quantitative PCR, droplet digital PCR, and the like.

In some embodiments, the method for assessing SCN10A expression levels is performed *in vitro* in cells that naturally express the SCN10A gene (e.g., hepatocytes) or cells that have been engineered to express SCN10A.

In other embodiments, the method for assessing SCN10A expression levels is performed *in vivo*. The antisense oligonucleotide or RNAi agent and any control antisense oligonucleotide or RNAi agent can be administered to an animal (e.g., a transgenic animal expressing the SCN10A gene or a non-human primate), and SCN10A mRNA or serum SCN10A protein levels are assessed in liver tissue harvested from the animal post-treatment. Alternatively or additionally, biomarkers or functional phenotypes associated with SCN10A expression can be assessed in the treated animal. For example, SCN10A protein is the expression product of SCN10A mRNA in serum or plasma. Therefore, the serum or plasma levels of SCN10A protein can be measured in an animal treated with an antisense oligonucleotide or RNAi agent of the invention to assess the functional efficacy of reducing SCN10A expression.

In certain embodiments, the expression of SCN10A in hepatocytes is reduced by the antisense oligonucleotide or RNAi agent of the invention by at least 40%, at least 45%, or at least 50%. In some embodiments, the expression of SCN10A in hepatocytes is reduced by the RNAi agent of the invention by at least 60%, at least 65%, at least 70%, at least 75%, at least 80%, or at least 85%. In other embodiments, the expression of SCN10A in hepatocytes is reduced by the antisense oligonucleotide or RNAi agent of the invention by about 90% or more, e.g., 91%, 92%, 93%, 94%, 95%, 96%, 97%, 98%, 99%, or more. The percentage reduction in SCN10A expression can be measured by any of the methods described herein, as well as other methods known in the art.

The present invention provides methods for reducing or inhibiting the expression of the SCN10A gene, thereby reducing or inhibiting the production of SCN10A protein, in a patient in need thereof, as well as methods for treating or preventing a disease or disorder associated with SCN10A expression or activity. A "disease or disorder associated with SCN10A expression" refers to a disease or disorder characterized by altered levels of SCN10A expression, or a condition where elevated levels of SCN10A expression are associated with an increased risk of developing the disease or disorder. In certain embodiments, the RNAi agents of the invention are particularly useful for treating or preventing metabolic diseases and reducing circulating levels of SCN10A.

Diseases and disorders associated with SCN10A expression that can be treated or prevented according to the methods of the invention include, but are not limited to, acute and chronic pain, such as traumatic and postoperative pain, nociceptive pain (e.g., pain caused by osteoporosis and rheumatoid arthritis), neuropathic pain (central types, such as post-stroke pain, spinal cord injury, migraine, HIV-associated neuropathic pain, etc.; peripheral types, such as postherpetic neuralgia and diabetic neuropathy), visceral pain (e.g., pain arising from pancreatitis, inflammatory bowel syndrome, etc.), and mixed pain (e.g., low back pain, cancer pain, fibromyalgia, etc.).

In certain embodiments, the present invention provides a method for reducing SCN10A expression in a patient in need thereof, comprising administering to the patient any antisense oligonucleotide or RNAi agent described herein. Preferably, the level of SCN10A expression in the patient's hepatocytes is reduced following administration of the antisense oligonucleotide or RNAi agent, compared to the level of SCN10A expression in a patient not receiving the antisense oligonucleotide or RNAi agent, or compared to the level of SCN10A expression in the patient prior to administration of the antisense oligonucleotide or RNAi agent. In some embodiments, after administration of the antisense oligonucleotide or RNAi agent of the invention, SCN10A expression in the patient is reduced by at least 30%, at least 35%, at least 40%, at least 45%, at least 50%, at least 55%, at least 60%, at least 65%, at least 70%, at least 75%, at least 80%, at least 85%, or at least 90%, e.g., 91%, 92%, 93%, 94%, 95%, 96%, 97%, 98%, or 99%. The percentage reduction in SCN10A expression can be measured by any of the methods described herein, as well as other methods known in the art. In certain embodiments, the percentage reduction in SCN10A expression is determined by assessing the level of SCN10A protein in the patient's serum or plasma according to the methods described herein.

In certain embodiments, a patient in need of reducing SCN10A expression is a patient diagnosed with or at risk of developing a disease mediated by aberrant expression levels of SCN10A. Accordingly, the present invention includes a method of treating or preventing a disease mediated by aberrant expression levels of SCN10A in a patient in need thereof by administering any antisense oligonucleotide or RNAi agent of the present invention. In some embodiments, the present invention includes use of any antisense oligonucleotide or RNAi agent described herein in the manufacture of a medicament for treating or preventing a disease mediated by aberrant expression levels of SCN10A in a patient in need thereof. In other embodiments, the present invention provides an SCN10A-targeting antisense oligonucleotide or RNAi agent for use in a method of treating or preventing a disease mediated by aberrant expression levels of SCN10A in a patient in need thereof. As described above, the disease mediated by aberrant expression levels of SCN10A is, for example, acute and chronic pain, such as traumatic and postoperative pain, nociceptive pain (e.g., pain caused by osteoporosis and rheumatoid arthritis), neuropathic pain (central types, such as post-stroke pain, spinal cord injury, migraine, HIV-associated neuropathic pain, etc.; peripheral types, such as postherpetic neuralgia and diabetic neuropathy), visceral pain (e.g., pain arising from pancreatitis, inflammatory bowel syndrome, etc.), and mixed pain (e.g., low back pain, cancer pain, fibromyalgia, etc.).

In certain other embodiments, a patient in need of reducing SCN10A expression is a patient with elevated levels of SCN10A. Accordingly, in some embodiments, the present invention provides a method of reducing SCN10A protein levels in a patient in need thereof by administering to the patient any antisense oligonucleotide or RNAi agent described herein. In some embodiments, the present invention includes use of any antisense oligonucleotide or RNAi agent described herein in the manufacture of a medicament for reducing SCN10A protein levels in a patient in need thereof. In other embodiments, the present invention provides an SCN10A-targeting antisense oligonucleotide or RNAi agent for use in a method of reducing SCN10A protein levels in a patient in need thereof.

### Sequence Listings

**Table 1. Oligonucleotides targeting SCN10A (naked sequences and exemplary modified sequences)**

| **Duplex** | **ASO 5'-3'** | **SED ID NO.** | **Naked Sequences (5'-3')** | **SED ID NO.** |
|---|---|---|---|---|
| 5008-2 | eT*eA*eG*eA*eA*m5dC*dT*dT*dG*dG*dG* m5dC*dA*dG*m5dC*eT*eG*eG*eT*eT | 21 | TAGAACTTGGGCAGCTG GTT | 3 |
| 5010-2 | eG*eT*eT*eG*eA*dA*dA*dG*dG*dA*m5dC*d T*dG*dA*dA*eT*eA*eG*m5eC*m5eC | 22 | GTTGAAAGGACTGAAT AGCC | 4 |
| 5011-2 | eA*eG*eA*m5eC*eA*m5dC*dT*dT*dT*dG*d A*dT*dG*dG*m5dC*m5eC*eG*eT*eT*m5eC | 23 | AGACACTTTGATGGCCG TTC | 5 |
| 5013-2 | eT*m5eC*eA*eG*eT*dT*m5dC*dG*dG*dG*dT *m5dC*dA*dT*dG*m5eC*eA*m5eC*eA*m5eC | 24 | TCAGTTCGGGTCATGCA CAC | 6 |
| 5014-2 | eA*eA*eG*eG*eT*m5dC*dA*dG*dT*dT*m5d C*dG*dG*dG*dT*m5eC*eA*eT*eG*m5eC | 25 | AAGGTCAGTTCGGGTCA TGC | 7 |
| 5017-2 | eA*m5eC*eA*eT*eA*dT*dG*m5dC*m5dC*dA *dG*dG*dG*dT*dA*eA*eT*eG*eA*m5eC | 26 | ACATATGCCAGGGTAAT GAC | 8 |
| 5026-2 | eT*eG*eA*m5eC*m5eC*dA*dG*dG*dT*dA*d G*dA*dA*dA*dG*eA*eT*m5eC*m5eC*m5eC | 27 | TGACCAGGTAGAAAGA TCCC | 9 |
| 5037-2 | eT*eA*eA*eT*eA*dG*dT*dA*dT*dG*dG*dG* dT*m5dC*dG*eA*eA*eG*eG*m5eC | 28 | TAATAGTATGGGTCGAA GGC | 10 |
| 5040-2 | eA*m5eC*eT*eG*eA*dG*dT*dT*dT*m5dC*m 5dC*dG*dA*dT*dG*eA*eT*m5eC*eT*eT | 29 | ACTGAGTTTCCGATGAT CTT | 11 |
| 5042-2 | eA*eT*eG*eT*eT*m5dC*dT*m5dC*dA*dA*dT *m5dC*m5dC*dA*m5dC*eT*m5eC*eT*m5eC* m5eC | 30 | ATGTTCTCAATCCACTC TCC | 12 |
| 5051-2 | eT*eA*eT*eT*m5eC*dA*m5dC*dA*dA*dT*dG *dA*dG*dG*dA*eA*eG*eT*m5eC*m5eC | 31 | TATTCACAATGAGGAA GTCC | 13 |
| 5054-2 | eT*eG*eT*eT*eG*dA*dG*dG*m5dC*dA*dG*d A*dT*dG*dA*eG*eG*eA*m5eC*m5eC | 32 | TGTTGAGGCAGATGAG GACC | 14 |
| 5059-2 | eA*eA*m5eC*eA*eT*dG*dT*m5dC*dG*dT*m 5dC*dG*dA*dT*dG*m5eC*m5eC*eA*eG*m5e C | 33 | AACATGTCGTCGATGCC AGC | 15 |
| 5060-2 | eT*eT*eG*eA*eA*m5dC*dA*dT*dG*dT*m5dC *dG*dT*m5dC*dG*eA*eT*eG*m5eC*m5eC | 34 | TTGAACATGTCGTCGAT GCC | 16 |
| 5069-2 | eA*eG*eA*eG*eT*dG*dG*dT*dT*dG*m5dC*d T*dA*dT*dT*eG*eG*eT*eT*m5eC | 35 | AGAGTGGTTGCTATTGG TTC | 17 |
| QS-172 | eA*eT*eG*eG*eT*m5dC*m5dC*dT*m5dC*m5 dC*m5dC*dT*dT*dT*dG*eT*eT*m5eC*eA*eG | 36 | ATGGTCCTCCCTTTGTT CAG | 18 |
| QS-173 | m5eC*m5eC*m5eC*eT*eT*dG*dA*dA*dG*dA *dG*dT*dT*dG*m5dC*eA*eG*m5eC*m5eC*m 5eC | 37 | CCCTTGAAGAGTTGCAG CCC | 19 |
| QS-174 | eG*eA*eA*m5eC*eT*dG*dG*dT*dT*dG*dA*d T*dT*dT*dT*eG*m5eC*m5eC*m5eC*eA | 38 | GAACTGGTTGATTTTGC CCA | 20 |
| e represents a 2'-MOE sugar moiety, d represents a 2'-deoxy sugar moiety, m5[e/d]C represents a 5-methylcytosine modification combined with either an e or d modification, and * represents a phosphorothioate modification. | | | | |

**Table 2. Nucleotide Unmodified SCN10A siRNA**

| **Duple x** | **SS** | **SS 5'-3'** | **SE Q ID NO** | **AS** | **AS 5'-3'** | **SEQ ID NO** |
|---|---|---|---|---|---|---|
| 51059 | 51059-S0 | ACAGCACACACCG GACAUU | 39 | 51059-A0 | AAUGUCCGGUGUGUGC UGUAG | 59 |
| 51069 | 51069-S0 | CCUUUCAACCUGA UCAGAA | 40 | 51069-A0 | UUCUGAUCAGGUUGAA AGGAC | 60 |
| 51071 | 51071-S0 | GAAGAACGGCCAU CAAAGU | 41 | 51071-A0 | ACUUUGAUGGCCGUUC UUCUG | 61 |
| 51078 | 51078-S0 | CGAACUGACCUUC CAGAGA | 42 | 51078-A0 | UCUCUGGAAGGUCAGU UCGGG | 62 |
| 51089 | 51089-S0 | GAUACUGGCAAGA GGAUUU | 43 | 51089-A0 | AAAUCCUCUUGCCAGU AUCUU | 63 |
| 51120 | 51120-S0 | ACACCAGCUUUGA UUCCUU | 44 | 51120-A0 | AAGGAAUCAAAGCUGG UGUAG | 64 |
| 51154 | 51154-S0 | ACCAGCGCAGGAU GUCUUU | 45 | 51154-A0 | AAAGACAUCCUGCGCU GGUUG | 65 |
| 51201 | 51201-S0 | CAGAAGAAGUGGA AUAUCU | 46 | 51201-A0 | AGAUAUUCCACUUCUU CUGGA | 66 |
| 51264 | 51264-S0 | GCUGGUUUGAGAG CUUCAU | 47 | 51264-A0 | AUGAAGCUCUCAAACC AGCUG | 67 |
| 51284 | 51284-S0 | GCUGGCUGGACUU CCUCAU | 48 | 51284-A0 | AUGAGGAAGUCCAGCC AGCAC | 68 |
| 51286 | 51286-S0 | GGACUUCCUCAUU GUGAAU | 49 | 51286-A0 | AUUCACAAUGAGGAAG UCCAG | 69 |
| 51329 | 51329-S0 | GCUGGAUGGACAU UAUGUA | 50 | 51329-A0 | UACAUAAUGUCCAUCC AGCCU | 70 |
| 51346 | 51346-S0 | GUCAUAAUUGACA ACUUCA | 51 | 51346-A0 | UGAAGUUGUCAAUUAU GACCC | 71 |
| 51350 | 51350-S0 | AGGACAUCUUCAU GACAGA | 52 | 51350-A0 | UCUGUCAUGAAGAUGU CCUGG | 72 |
| 51370 | 51370-S0 | CCAUGAUGGUGGA GACUGA | 53 | 51370-A0 | UCAGUCUCCACCAUCA UGGUG | 73 |
| 51407 | 51407-S0 | CUUGUCAUGUUCA UCUACU | 54 | 51407-A0 | AGUAGAUGAACAUGAC AAGGA | 74 |
| 51415 | 51415-S0 | UCGACGACAUGUU CAACUU | 55 | 51415-A0 | AAGUUGAACAUGUCGU CGAUG | 75 |
| 51417 | 51417-S0 | UGUUCAACUUCCA GACCUU | 56 | 51417-A0 | AAGGUCUGGAAGUUGA ACAUG | 76 |
| 51435 | 51435-S0 | UGAUUCUGGAGAA CUUCAA | 57 | 51435-A0 | UUGAAGUUCUCCAGAA UCACU | 77 |
| 51441 | 51441-S0 | ACGACUUUGACAU GUUCUA | 58 | 51441-A0 | UAGAACAUGUCAAAGU CGUCC | 78 |

Human SCN10A, mRNA (SEQ ID NO. 1, NCBI Accession No. NM_006514.4)

1 ttcttgcttc acacactagc ctcttggcta gagaacagac atcagatgga gtttcttctg
61 gctatgcctg aatgttaagc tgaacgtatg ttccaggagc tcgtggtctc cagtagaggc
121 aatctgggat agaagagaag atatttctta cgtagaagac aagcaagatt gagcagcaaa
181 gagtataaat acttcctgaa gaagaatgag aagatggaat tccccattgg atccctcgaa
241 actaacaact tccgtcgctt tactccggag tcactggtgg agatagagaa gcaaattgct
301 gccaagcagg gaacaaagaa agccagagag aagcataggg agcagaagga ccaagaagag
361 aagcctcggc cccagctgga cttgaaagcc tgcaaccagc tgcccaagtt ctatggtgag
421 ctcccagcag aactgatcgg ggagcccctg gaggatctag atccgttcta cagcacacac
481 cggacattta tggtgctgaa caaagggagg accatttccc ggtttagtgc cactcgggcc
541 ctgtggctat tcagtccttt caacctgatc agaagaacgg ccatcaaagt gtctgtccac

601 tcgtggttca gtttatttat tacggtcact attttggtta attgtgtgtg catgacccga
661 actgaccttc cagagaaaat tgaatatgtc ttcactgtca tttacacctt tgaagccttg
721 ataaagatac tggcaagagg attttgtcta aatgagttca cgtacctgag agatccttgg
781 aactggctgg attttagcgt cattaccctg gcatatgttg gcacagcaat agatctccgt
841 gggatctcag gcctgcggac attcagagtt cttagagcat taaaaacagt ttctgtgatc
901 ccaggcctga aggtcattgt gggggccctg attcactcag tgaagaaact ggctgatgtg
961 accatcctca ccatcttctg cctaagtgtt tttgccttgg tggggctgca actcttcaag
1021 ggcaacctca aaaataaatg tgtcaagaat gacatggctg tcaatgagac aaccaactac
1081 tcatctcaca gaaaaccaga tatctacata aataagcgag gcacttctga ccccttactg
1141 tgtggcaatg gatctgactc aggccactgc cctgatggtt atatctgcct taaaacttct
1201 gacaacccgg attttaacta caccagcttt gattcctttg cttgggcttt cctctcactg
1261 ttccgcctca tgacacagga ttcctgggaa cgcctctacc agcagaccct gaggacttct
1321 gggaaaatct atatgatctt ttttgtgctc gtaatcttcc tgggatcttt ctacctggtc
1381 aacttgatct tggctgtagt caccatggcg tatgaggagc agaaccaggc aaccactgat
1441 gaaattgaag caaaggagaa gaagttccag gaggccctcg agatgctccg gaaggagcag
1501 gaggtgctag cagcactagg gattgacaca acctctctcc actcccacaa tggatcacct
1561 ttaacctcca aaaatgccag tgagagaagg catagaataa agccaagagt gtcagagggc
1621 tccacagaag acaacaaatc accccgctct gatccttaca accagcgcag gatgtctttt
1681 ctaggcctcg cctctggaaa acgccgggct agtcatggca gtgtgttcca tttccggtcc
1741 cctggccgag atatctcact ccctgaggga gtcacagatg atggagtctt tcctggagac
1801 cacgaaagcc atcggggctc tctgctgctg ggtgggggtg ctggccagca aggccccctc
1861 cctagaagcc ctcttcctca acccagcaac cctgactcca ggcatggaga agatgaacac
1921 caaccgccgc ccactagtga gcttgcccct ggagctgtcg atgtctcggc attcgatgca
1981 ggacaaaaga agactttctt gtcagcagaa tacttagatg aacctttccg ggcccaaagg
2041 gcaatgagtg ttgtcagtat cataacctcc gtccttgagg aactcgagga gtctgaacag
2101 aagtgcccac cctgcttgac cagcttgtct cagaagtatc tgatctggga ttgctgcccc
2161 atgtgggtga agctcaagac aattctcttt gggcttgtga cggatccctt tgcagagctc
2221 accatcacct tgtgcatcgt ggtgaacacc atcttcatgg ccatggagca ccatggcatg
2281 agccctacct tcgaagccat gctccagata ggcaacatcg tctttaccat atttttact
2341 gctgaaatgg tcttcaaaat cattgccttc gacccatact attatttcca gaagaagtgg
2401 aatatctttg actgcatcat cgtcactgtg agtctgctag agctgggcgt ggccaagaag
2461 ggaagcctgt ctgtgctgcg gagcttccgc ttgctgcgcg tattcaagct ggccaaatcc
2521 tggcccacct taaacacact catcaagatc atcggaaact cagtgggggc actggggaac
2581 ctcaccatca tcctggccat cattgtcttt gtctttgctc tggttggcaa gcagctccta
2641 ggggaaaact accgtaacaa ccgaaaaaat atctccgcgc cccatgaaga ctggccccgc
2701 tggcacatgc acgacttctt ccactctttc ctcattgtct tccgtatcct ctgtggagag
2761 tggattgaga acatgtgggc ctgcatggaa gttggccaaa aatccatatg cctcatcctt
2821 ttcttgacgg tgatggtgct agggaacctg gtggtgctta acctgttcat cgccctgcta
2881 ttgaactctt tcagtgctga caacctcaca gccccggagg acgatgggga ggtgaacaac
2941 ctgcaggtgg ccctggcacg gatccaggtc tttggccatc gtaccaaaca ggctctttgc
3001 agcttcttca gcaggtcctg cccattcccc cagcccaagg cagagcctga gctggtggtg
3061 aaactcccac tctccagctc caaggctgag aaccacattg ctgccaacac tgccaggggg
3121 agctctggag ggctccaagc tcccagaggc cccagggatg agcacagtga cttcatcgct
3181 aatccgactg tgtgggtctc tgtgcccatt gctgagggtg aatctgatct tgatgacttg
3241 gaggatgatg gtggggaaga tgctcagagc ttccagcagg aagtgatccc caaaggacag
3301 caggagcagc tgcagcaagt cgagaggtgt ggggaccacc tgacacccag gagcccaggc
3361 actggaacat cttctgagga cctggctcca tccctgggtg agacgtggaa agatgagtct
3421 gttcctcagg tccctgctga gggagtggac gacacaagct cctctgaggg cagcacggtg
3481 gactgcctag atcctgagga aatcctgagg aagatccctg agctggcaga tgacctggaa
3541 gaaccagatg actgcttcac agaaggatgc attcgccact gtccctgctg caaactggat
3601 accaccaaga gtccatggga tgtgggctgg caggtgcgca agacttgcta ccgtatcgtg
3661 gagcacagct ggtttgagag cttcatcatc ttcatgatcc tgctcagcag tggatctctg

3721 gcctttgaag actattacct ggaccagaag cccacggtga aagctttgct ggagtacact
3781 gacagggtct tcacctttat ctttgtgttc gagatgctgc ttaagtgggt ggcctatggc
3841 ttcaaaaagt acttcaccaa tgcctggtgc tggctggact tcctcattgt gaatatctca
3901 ctgataagtc tcacagcgaa gattctggaa tattctgaag tggctcccat caaagccctt
3961 cgaacccttc gcgctctgcg gccactgcgg gctctttctc gatttgaagg catgcgggtg
4021 gtggtggatg ccctggtggg cgccatccca tccatcatga atgtcctcct cgtctgcctc
4081 atcttctggc tcatcttcag catcatgggt gtgaacctct tcgcagggaa gttttggagg
4141 tgcatcaact ataccgatgg agagttttcc cttgtacctt tgtcgattgt gaataacaag
4201 tctgactgca agattcaaaa ctccactggc agcttcttct gggtcaatgt gaaagtcaac
4261 tttgataatg ttgcaatggg ttaccttgca cttctgcagg tggcaacctt taaaggctgg
4321 atggacatta tgtatgcagc tgttgattcc cgggaggtca acatgcaacc caagtgggag
4381 gacaacgtgt acatgtattt gtactttgtc atcttcatca tttttggagg cttcttcaca
4441 ctgaatctct ttgttggggt cataattgac aacttcaatc aacagaaaaa aaagttaggg
4501 ggccaggaca tcttcatgac agaggagcag aagaaatact acaatgccat gaagaagttg
4561 ggctccaaga agccccagaa gcccatccca cggcccctga acaagttcca gggttttgtc
4621 tttgacatcg tgaccagaca agcttttgac atcaccatca tggtcctcat ctgcctcaac
4681 atgatcacca tgatggtgga gactgatgac caaagtgaag aaaagacgaa aattctgggc
4741 aaaatcaacc agttctttgt ggccgtcttc acaggcgaat gtgtcatgaa gatgttcgct
4801 ttgaggcagt actacttcac aaatggctgg aatgtgtttg acttcattgt ggtggttctc
4861 tccattgcga gcctgatttt ttctgcaatt cttaagtcac ttcaaagtta cttctcccca
4921 acgctcttca gagtcatccg cctggcccga attggccgca tcctcagact gatccgagcg
4981 gccaagggga tccgcacact gctctttgcc ctcatgatgt ccctgcctgc cctcttcaac
5041 atcgggctgt tgctattcct tgtcatgttc atctactcta tcttcggtat gtccagcttt
5101 ccccatgtga ggtgggaggc tggcatcgac gacatgttca acttccagac cttcgccaac
5161 agcatgctgt gcctcttcca gattaccacg tcggccggct gggatggcct cctcagcccc
5221 atcctcaaca cagggccccc ctactgtgac cccaatctgc ccaacagcaa tggcaccaga
5281 ggggactgtg ggagcccagc cgtaggcatc atcttcttca ccacctacat catcatctcc
5341 ttcctcatca tggtcaacat gtacattgca gtgattctgg agaacttcaa tgtggccacg
5401 gaggagagca ctgagcccct gagtgaggac gactttgaca tgttctatga gacctgggag
5461 aagtttgacc cagaggccac tcagtttatt accttttctg ctctctcgga ctttgcagac
5521 actctctctg gtcccctgag aatcccaaaa cccaatcgaa atatactgat ccagatggac
5581 ctgcctttgg tccctggaga taagatccac tgcttggaca tcctttttgc tttcaccaag
5641 aatgtcctag gagaatccgg ggagttggat tctctgaagg caaatatgga ggagaagttt
5701 atggcaacta atctttcaaa atcatcctat gaaccaatag caaccactct ccgatggaag
5761 caagaagaca tttcagccac tgtcattcaa aaggcctatc ggagctatgt gctgcaccgc
5821 tccatggcac tctctaacac cccatgtgtg cccagagctg aggaggaggc tgcatcactc
5881 ccagatgaag gttttgttgc attcacagca aatgaaaatt gtgtactccc agacaaatct
5941 gaaactgctt ctgccacatc attcccaccg tcctatgaga gtgtcactag aggccttagt
6001 gatagagtca acatgaggac atctagctca atacaaaatg aagatgaagc caccagtatg
6061 gagctgattg cccctgggcc ctagtgagaa cactccagcc tggatatgtt cagttatgat
6121 gcgtccttct gctctgtgtt aactctttcc cattgcagat cacacccagc tacagcctca
6181 ccaatgcatg ccactggtca caatgtcaga actgggcagg gaatgcaact ggtaaccacc
6241 tttgaaaaaa ccatcataca caaataggag tcagaagcta agagcacttc cactgtgatt
6301 tcccttctga atttcaatat cagatcatgg gttctcttac tctccagaaa atatcccttg
6361 tccttttatt tttgttgtaa tcagattata tttactgaga caaacttctc aggaccagaa
6421 cttccaaaga tatagaacaa gaacattact gaaggtccaa ggtagtcctc tgtggagcac
6481 catacagaga ttggcaatat tatttaggat tttgcatgac tgcatgtgag agctgtcgga
6541 caactgctct gacccagggt aacacctgtg gcctatgtca tgaaaagcct ttgagatatc
6601 cattaaaatt aatattttta aatgta

Cynomolgus Monkey SCN10A mRNA (SEQ ID NO. 2, NCBI Accession No. XM_005546684.3)

1 atgagaagat ggaattcccc attggatccc tcggaactaa caactttcgt cgctttactc

61 cggagtcact ggtggagata gagaagcaaa ttgctgccaa gcaggcagca aagaaagcca
121 gagagaagca cagggagcag aaggaccaag aagagaagac tcggccacag ctagacttga
181 aagcctgcaa ccagctgccc aagttctatg gtgagctccc agcagaactg atcggggagc
241 ccctggagga tctagacacc ttctacagca cacaccggac atttatggtg ctgaacaaag
301 ggaggaccat tttccggttt agtgccactc gggccctgtg gctattcagt cctttcaacc
361 tgatcagaag aacggccatc aaagtgtctg tccactcata tcctttgtgg ttcagtttat
421 ttattacggt cactattttg gttaattgtg tgtgcatgac ccgaactgac cttccagaga
481 aaattgagta tgtcttcacc gtcatttaca cctttgaagc cttgataaag atactggcaa
541 gaggattttg tctaaatgag ttcacgtacc tgagagatcc ttggaactgg ctggatttta
601 gcgtcattac cctggcatat gttggcacag caatagatct ccgtgggatc tcaggcctcc
661 ggacattcag agttcttaga gcattaaaaa cagtttctgt gatcccaggc ctgaaagtca
721 ttgtgggggc cctgattaac tcagtgaaga aactggctga tgtgaccatc ctcaccatct
781 tctgcctaag tgtttttgcc ttggtggggc tgcaactctt caagggcaac ctcaagaata
841 aatgtgtcaa gaatgatatg gctgtcaatg agacaaccaa ctactcatct cacagaaaac
901 cagatatcta cataaacaag cgaggcactt ctgatccctt actgtgtggc aatggatctg
961 actcaggcca ctgtcctgat ggttatatct gccttaaaac ttctgacaac ccggatttta
1021 actacaccag ctttgattcc tttgcttggg ctttcctctc actgttccgc ctcatgacac
1081 aggattcctg ggaacgcctc taccagcaga ccctgagggc ttctgggaaa atctatatga
1141 tcttttttgt gctcgtaatc ttcctgggat ctttctacct ggtcaacttg atcttggctg
1201 tagtcaccat ggcatatgag gagcagaaca aggcaaccat tgatgaaatt gaagcaaagg
1261 agaagttgtt ccaggagacc ctcgaaaagc tccggaagga gcaggaggtg ctggcagcgc
1321 tggggattga cacaacctct ctccactccc acaacggatc acctttaacc tccaaaaatg
1381 ccagcgagag aaggcacaga ataaagtcaa gagtgtcaga gggctccaca gaagacaaca
1441 aatcaccccg ttctgatcct tacaaccagc gcaggatgtc ttttctaggc ctcgcctctg
1501 gaaaacgccg ggctagccat ggcagtgtgt tccatttccg gtcccccggc cgagatatct
1561 cactccctga ggtagtcaca gatgatggag tctttcctgg agaccatgaa agccatcggg
1621 gctccctgct gctgggtggg agtgctggcc agcaaggccc cctccctaga agccctcttc
1681 ctcaacccag caaccctgac tccaggcatg gagaagatga acacccactg ccacccacta
1741 gtgagcttgc ccctggagct gtcgaagtct tggcattcga tgcaggacaa aagaagactt
1801 tcttgtcagc agaatactta gatgaacctt tccgagccca aagggcaatg agtgttgtca
1861 gtatcataac ctccgtcctt gaggagctcg aggagtctga acagaagtgc ccaccctgct
1921 tgaccagctt ggctcagaag tatctgatct gggattgctg ccctatgtgg gtgaagctca
1981 agacaattct ctttgggctt gtgacggacc cctttgcaga gctcaccatc accttgtgca
2041 tcgtggtgaa caccatcttc atggccatgg agcatcatgg catgagccct accttcgaag
2101 ccatgcttca gataggcaac attgtcttta ccatattttt tactgctgaa atggtcttca
2161 aaatcattgc cttcgaccca tactattatt tccagaagaa gtggaatatc tttgactgca
2221 tcatcgttac tgtgagtctg ctagagctgg gcgtggccaa gaagggaagc ctgtctgtgc
2281 tgcggagctt ccgcttgctg cgcgtcttca agctggccaa gtcctggccc accttaaaca
2341 cgctcatcaa gatcatcgga aactcagtgg gggcactggg gaacctcacc atcatcctgg
2401 ccatcattgt ctttgtcttt gctctggttg gcaagcagct cctaggggaa aactaccgta
2461 ataaccgaaa aaatatctcc gcgccccatg aagactggcc ccgctggcac atgcacgact
2521 tcttccactc tttcctcatt gtcttccgca ttctctgtgg agagtggatt gagaacatgt
2581 gggcctgcat ggaagtcggc cagaaatcca tatgcctcat ccttttcttg acggtgatgg
2641 tgctggggaa cctggtggtg cttaacctgt tcatcgccct gctattgaac tctttcagtg
2701 ctgacaacct cacagctccg gaggatgatg gggaggtgaa caacctgcag gtggccctgg
2761 cacggatcca ggtctttggc catcgtatca agcaggttct ttgcagcttc ttcagcaggc
2821 cctgcccatt cccccggccc aaggcagagc cggagctggt ggtgaaactc ccactctcca
2881 gctccaaagc tgagaaccac attgctgcca acaatgccga gggaagctct ggagggctcc
2941 aagctcccag acgccccagg gatgagcaca gtgacttcat cgctaatcct actgtgtggg
3001 tctctgtgcc cattgctgag ggcgaatccg atcttgatga cttggaggat gatggtgagg
3061 aagatgctca gagcgcccaa caagaagtga tccccaaagg acagcaggag cagctgcagc
3121 aagttgagag gtgtgaggac cacctgacag tcaggagccc aggcactgga acatcttctg

3181 aggacctggc tccatacctg ggtgagacgt ggaaagatga gtctgttcct caggcccctg
3241 cagagggagt ggatgacaca agctcctccg agggcagcac ggtggactgc ccagatcctg
3301 aggagatcct gaggaagatc cctgagctgg cggatgacct agaagagcca gatgactgct
3361 tcacagaagg atgcattcgc cactgtccct gctgcaaagt ggataccacc aagagtccat
3421 gggacatggg ctggcaggtg cgcaagactt gctaccgtat tgtggagcac agctggtttg
3481 aaagcttcat catcttcatg atcctgctca gcagtggatc tctggccttt gaagactatt
3541 acctggacca gaagcccacg gtgaaagctt tgctggaata cactgacagg gtcttcacct
3601 ttatctttgt gttcgagatg ctgcttaagt gggtggccta tggcttcaaa aagtacttca
3661 ccaatgcctg gtgctggctg gacttcctca ttgtgaatat ctcactgaca agtctcacag
3721 caaagattct ggaatattct gaagtggctc ccatcaaagc cctacgaacc cttcgtgctc
3781 tgcggccact gcgggctctg tctcgatttg aaggcatgcg ggtggtggtg gatgccctgg
3841 tgggcgccat cccgtccatc atgaacgtcc tcctcgtctg cctcatcttc tggctaatct
3901 tcagcatcat gggtgtgaac ctcttcgcag ggaagttttg gaggtgcatc aactacaccg
3961 atggagagtt ttctcttgta cctttgtcga ttgtgaataa caagtctgac tgcaagattc
4021 aaaactccac cggcagcttc ttctgggtca atgtgaaagt caactttgat aatgttgcaa
4081 tgggttacct tgcacttctg caggtggcaa cctttaaagg ctggatggac attatgtatg
4141 cagctgtcga ttcccgggag gtcaacatgc aacccaagtg ggaggacaac gtgtacatgt
4201 atttgtactt cgtcatcttc atcattttcg gaggcttctt cacactgaat ctctttgttg
4261 gggtcataat tgacaacttc aatcaacaga aaaaaaagtt agggggccag gacatcttta
4321 tgacagagga gcagaagaag tactacaatg ccatgaagaa gctgggctcc aagaagcccc
4381 agaagcccat cccacggccc ctgaacaagt tccagggttt tgtctttgac atcgtgacca
4441 gacaagcttt tgacatcacc atcatggtcc tcatctgcct caacatgatc accatgatgg
4501 tggagactga tgaccagagt gaagaaaaga cgaaaattct gggcaaaatc aaccagttct
4561 ttgtggccgt cttcacgggc gaatgtgtca tgaagatgtt cgctttgagg cagtactact
4621 tcaccaatgg ctggaatgtg tttgacttca ttgtcgtggt tctctccatt gcgagcctgg
4681 ttttttctgc aattcttaag tcgcttcaaa attacttctc cccgacgctc ttccgagtca
4741 tccgcctggc ccgaatcggc cgcatcctca gactgatccg agcggccaag gggatccgca
4801 cactactctt tgccctcatg atgtccctgc ctgccctctt caacatcggg ctactgctct
4861 tccttgtcat gttcatctac tccatcttcg gcatgtccag ctttccccat gtgaggtggg
4921 aggctggcat cgacgacatg ttcaacttcc agaccttcgc caacagtatg ctgtgcctct
4981 tccagatcac cacgtcagcc ggctgggatg gcctcctcag ccccatcctc aacacggggc
5041 ccccctactg tgaccccaat ctgcccaaca gcaatggcac cagaggggac tgtgggagcc
5101 cagctgtggg catcatcttc ttcaccactt acatcattat ctccttcctc atcgtggtca
5161 acatgtacat tgcagtgatt ctggagaact tcaacgtggc cacggaggag agcaccgagc
5221 ccctgagtga ggacgacttt gacatgttct atgagacttg ggagaagttt gacccagagg
5281 ctactcagtt tattaccttt tctgctctct cagactttgc agacactctc tctggtcccc
5341 tgagaatccc aaaacccaat cgaaatatac tgatccagat ggacctgcct ttggtccctg
5401 gagacaagat ccactgcttg gacatccttt ttgctttcac caagaatgtc ctaggagagt
5461 ccggggagtt ggattctctg aaggcaaata tggaggagaa atttatggca actaatcttt
5521 caaagtcatc ctatgaacca atagcaacca ctctccgatg gaagcaagaa gacatttcag
5581 ccactgtcat tcaaaaggcc tatcggagct atgtgctgca ccgctccatg gcactctcca
5641 acaccctaca tgtgcccaga gctgaggagg aggctgcatc actcccagat gaagcctttg
5701 ttgcattcac agcaaatgaa aattgtgtac tgccagacaa atctgaaact gcatctgcca
5761 catctttccc accatcctat gagagtgtca ctagaggcct tagtgataga gtcaacatga
5821 ggacatctag ctcaatacaa aatgaagatg aagccaccag tacagaggtg actgcccctg
5881 ggccctagtg aggacactcc

### Examples

### Experiment Methods and Materials

### Naked Sequences Screening

The ASOs and siRNAs were designed based on the full-length mRNA sequence of SCN10A (NM_006514.4), and all sequences were derived from the NCBI gene database. All ASOs and siRNAs were designed to ensure homology with the sequences of human (Gene ID: 6336, NM_006514.4, SEQ ID NO. 1) and cynomolgus monkey (Gene ID:102122089, XM_005546684.3, SEQ ID NO. 2).

### Synthesis of Oligonucleotides

The synthesis method for the oligonucleotides is similar to the conventional phosphoramidite solid-phase synthesis method (all were synthesized by Suzhou Biosyntech Co., Ltd.), which includes four steps: deprotection, coupling, capping, and oxidation or sulfurization. The solid support was used as the starting cycle, and nucleoside monomers were sequentially linked in the 3' to 5' direction. The nucleoside phosphoramidite monomer raw materials, including 2'-OTBDMS, 2'-F RNA, 2'-OMe RNA, etc., were purchased from Hongene Biotech Corporation. After completion of solid-phase synthesis, the solid support was transferred to a centrifuge tube, and a 3:1 mixture of 28% ammonium hydroxide and ethanol was added to soak the support at 50 °C for 16 hours to cleave the oligonucleotides from the solid support into solution. After centrifugation, the supernatant was transferred to another centrifuge tube, concentrated to dryness, and then redissolved in deionized water. Purification was performed using a C18 reversed-phase column with 0.1 M TEAA and acetonitrile as the mobile phase. The target oligonucleotide was collected, lyophilized, identified as the desired product by LC-MS, and quantified by UV absorbance at 260 nm. The siRNA was obtained by annealing the sense strand and the antisense strand.

### Cell Lines and Reagents

SCN10A-overexpressing CHO cells were cultured in DMEM medium (Invitrogen, catalog number 11965118) supplemented with 10% fetal bovine serum (ExCellBio, catalog number FSP500), 1% penicillin-streptomycin (HyClone, catalog number SV30010), 1% non-essential amino acids (Gibco, catalog number 11140-050), 1% glutamine (Gibco, catalog number 35050-061), and 400 µg/mL G418 (Gibco, catalog number 10131-027).

| Cell Line | Reference Sequence | Sequence Length for Cell Line Construction (CDS) |
|---|---|---|
| CHO | NCBI AF117907 | 5874bp |

The main reagents used in this experiment included Lipofectamine^{™} RNAiMAX Transfection Reagent (Invitrogen, catalog number 13778150), HiScript III RT SuperMix for qPCR (Vazyme, catalog number R323-01), RNA extraction kit (Qiagen, catalog number 74182), AceQ Universal U+ Probe Master Mix V2 (Vazyme, catalog number Q513-03), GAPDH and SCN10A primer-probe sets synthesized by Suzhou Genewiz Biotechnology Co., Ltd., and 96-well plates (Costar 3599).

### Transfection of ASO/siRNA Compounds

On day 1, according to the ratio (RNAiMAX Transfection Reagent : Opti-MEM = 0.3 : 4.7), the mixture of the two was prepared as needed and incubated at room temperature for 15 minutes. For each well of cells, a certain amount of the diluted compound was added to an equal volume of the RNAiMAX-Opti-MEM mixture, mixed, and incubated at room temperature for 15 minutes. CHO cells were washed with DPBS and then digested with trypsin, and the cell density was adjusted to 2.22 × 10^5/mL. While seeding the cells, 10 µL of the Opti-MEM-RNAiMAX-compound mixture was added to the cell culture plate, and the cells were seeded into a 96-well plate at a density of 20,000 cells per well (90 µL/well), with a final culture volume of 100 µL per well. The cells were cultured in a 5% CO2 incubator at 37 °C for 24 hours.

### RNA Extraction and Reverse Transfection

RNA was extracted according to the instructions of the RNA extraction kit (Qiagen, catalog number 74182), and the RNA was reverse-transcribed into cDNA according to the instructions of the HiScript III RT SuperMix for qPCR (Vazyme, catalog number R323-01).

### QPCR Detection and Data Analysis

The target gene cDNA was detected by qPCR, and the qPCR reaction mix was prepared according to the formulation shown in Table 3. GAPDH was used as an internal reference gene, and the qPCR was performed in a 384-well plate. The qPCR thermal cycling program was as follows: heating at 95 °C for 10 minutes, followed by 40 cycles of heating at 95 °C for 15 seconds and then at 60 °C for 1 minute.

**Table 3. RT-PCR reaction system**

| **Components** | **Concentrations** | **Volume (µL) per well** |
|---|---|---|
| FastStart Universal Probe Mast | 2× | 5 |
| SCN10A/GAPDH forward priMer | 10 µM | 0.4 |
| SCN10A/GAPDH reverse priMer | 10 µM | 0.4 |
| SCN10A/GAPDH probe | 10 µM | 0.2 |
| RNase-free Water | / | 2 |
| Total | / | 8 |

### Data Analysis (ΔΔCT method (CT difference comparison method))

In this method, an internal reference gene, GAPDH, was introduced, as it is expressed in all cells, its product is essential for cell survival, and its expression level or genomic copy number in cells is constant and is less affected by environmental factors. Following qRT-PCR, the CT value of the internal reference gene was recorded simultaneously, which was referred to as Ct(GAPDH), and the CT value of the sample was referred to as Ct(sample).$Δ Ct \left(sample\right) = Ct \left(sample\right) - Ct \left(GAPDH\right)$$Δ Ct \left(control\right) = Ct \left(control\right) - Ct \left(GAPDH\right)$$ΔΔ Ct = Δ Ct \left(sample\right) - Δ Ct \left(control\right)$$Relative gene expression level = 2 - ΔΔ Ct$ 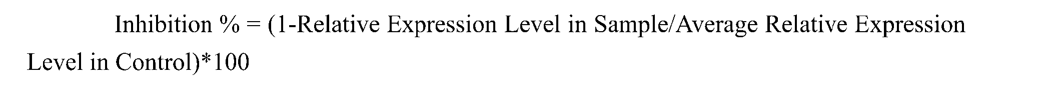 Inhibition % = (1-Relative Expression Level in Sample/Average Relative Expression Level in Control)*100

### Example 2. In Vitro Functional Evaluation of Modified ASO Sequences

To evaluate the *in vitro* inhibitory activity of the modified ASO compounds against SCN10A mRNA, the designed ASO compounds were subjected to *in vitro* functional evaluation, and the results were presented in Table 4.

**Table 4. In vitro inhibitory activity of ASO compounds against SCN10A mRNA in the CHO cell line.**

| **Compoun d ID** | **Average Inhibition %** | | | **SD** | | |
|---|---|---|---|---|---|---|
| | 100 nM | 50 nM | 10 nM | 100 nM | 50 nM | 10 nM |
| 5008-2 | 65.61 | 64.20 | 58.73 | 3.77 | 0.18 | 1.51 |
| 5010-2 | 62.44 | 57.52 | 40.43 | 1.13 | 1.89 | 10.17 |
| 5011-2 | 61.86 | 59.47 | 29.29 | 7.27 | 1.47 | 19.47 |
| 5013-2 | 35.35 | 44.39 | 29.32 | 8.40 | 3.78 | 4.63 |
| 5014-2 | 57.33 | 50.06 | 27.75 | 7.44 | 6.01 | 4.64 |
| 5017-2 | 52.48 | 46.15 | 10.34 | 2.89 | 5.63 | 15.15 |
| 5026-2 | 42.73 | 46.02 | 29.53 | 9.87 | 6.81 | 7.85 |
| 5037-2 | 56.47 | 50.40 | 34.14 | 4.77 | 4.02 | 4.40 |
| 5040-2 | 59.19 | 57.54 | 32.59 | 0.23 | 0.12 | 11.97 |
| 5042-2 | 37.16 | - | 31.08 | 7.89 | - | 9.57 |
| 5051-2 | 37.45 | - | 35.95 | 8.49 | - | 4.25 |
| 5054-2 | 34.17 | - | 37.19 | 8.39 | - | 5.03 |
| 5059-2 | 54.20 | - | 19.72 | 5.67 | - | 16.29 |
| 5060-2 | 40.24 | - | 21.28 | 5.63 | - | 6.03 |
| 5069-2 | 42.49 | - | 32.94 | 0.63 | - | 5.02 |
| QS-172 | 48.94 | - | 35.07 | 0.30 | - | 6.43 |
| QS-173 | 58.36 | - | 51.87 | 0.63 | - | 2.59 |
| QS-174 | 43.48 | - | 15.60 | 14.90 | - | 17.50 |

It was demonstrated that the ASOs provided by the present invention were capable of effectively reducing SCN 10A mRNA levels in the CHO cell line.

### Example 3. In vitro Functional Evaluation of Unmodified siRNA Sequences

To evaluate the *in vitro* inhibitory activity of the unmodified siRNAs against SCN10A mRNA in the CHO cell line, the designed siRNAs were subjected to *in vitro* functional evaluation, and the results were shown in Table 5.

**Table 5. In vitro inhibitory activity of siRNA compounds against SCN10A mRNA in the CHO cell line.**

| **Compound ID** | **Average Inhibition %** | | **SD** | |
|---|---|---|---|---|
| | 10 nM | 1 nM | 10 nM | 1 nM |
| 51059 | 53.55 | 37.80 | 5.63 | 9.12 |
| 51069 | 70.00 | 65.23 | 1.55 | 2.46 |
| 51071 | 63.48 | 60.02 | 1.93 | 1.30 |
| 51078 | 65.74 | 64.31 | 5.03 | 11.77 |
| 51089 | 67.12 | 61.25 | 4.14 | 5.29 |
| 51120 | 70.71 | 66.55 | 3.32 | 3.79 |
| 51154 | 62.70 | 52.16 | 3.96 | 12.60 |
| 51201 | 68.51 | 65.39 | 6.31 | 2.01 |
| 51264 | 65.39 | 64.92 | 2.26 | 3.51 |
| 51284 | 60.76 | 66.37 | 2.45 | 6.34 |
| 51286 | 68.89 | 68.67 | 1.87 | 1.28 |
| 51329 | 66.96 | 67.96 | 3.75 | 3.77 |
| 51346 | 67.79 | 60.99 | 11.48 | 0.65 |
| 51350 | 59.56 | 50.39 | 1.82 | 2.37 |
| 51370 | 49.16 | 27.80 | 3.33 | 3.66 |
| 51407 | 72.30 | 64.00 | 9.18 | 1.04 |
| 51415 | 66.10 | 61.68 | 7.77 | 2.00 |
| 51417 | 69.61 | 60.47 | 5.62 | 2.32 |
| 51435 | 69.82 | 59.58 | 8.74 | 8.17 |
| 51441 | 72.84 | 58.00 | 7.48 | 1.70 |

It was demonstrated that the siRNAs provided by the present invention were capable of effectively reducing SCN10A mRNA levels in the CHO cell line

## Claims

1. An antisense oligonucleotide comprising an oligonucleotide consisting of 12 to 30 contiguous nucleotides, wherein the oligonucleotide comprises at least 8, at least 9, at least 10, at least 11, at least 12, at least 13, at least 14, at least 15, at least 16, at least 17, at least 18, at least 19, or at least 20 contiguous nucleotides of any one of the nucleotide sequences set forth in SEQ ID NOs: 3 to 20.

2. The antisense oligonucleotide according to claim 1, wherein when measured over the entire nucleotide sequence of the antisense oligonucleotide, the antisense oligonucleotide has a nucleotide sequence that is at least 80%, at least 85%, at least 90%, at least 95% or 100% complementary to the nucleotide sequence set forth in SEQ ID NO. 1 and/or SEQ ID NO. 2.

3. The antisense oligonucleotide according to claim 1 or 2, wherein antisense oligonucleotide comprises at least one modification selected from a modified sugar moiety, a modified nucleobase moiety, and a modified internucleoside linkage; preferably, the antisense oligonucleotide comprises one or more of the following modifications:
(i) at least one bicyclic sugar moiety, wherein the bicyclic sugar moiety preferably has a 4'-2' bridge, and the 4'-2' bridge is preferably cEt or LNA;
(ii) at least one non-bicyclic modified sugar moiety, preferably selected from 2'-deoxy, 2'-F, 2'-MOE, and 2'-OMe sugar moieties;
(iii) a modified nucleoside, preferably comprising 5-methylcytosine (m5C); and
(iv) at least one sugar surrogate, preferably selected from POM, PNA, THP, and F-HNA.

4. The antisense oligonucleotide according to claim 3, wherein the antisense oligonucleotide comprises a gapmer; preferably, the gapmer comprises: (a) a 5' region consisting of 1 to 6 linked 5' region nucleotides; (b) a central region consisting of 6 to 10 linked central region nucleotides; and (c) a 3' region consisting of 1 to 6 linked 3' region nucleotides; wherein each of the nucleotides in the 5' region and the 3' region comprises a sugar moiety selected from 2'-MOE, LNA, and cEt modified sugar moieties, and at least 6 of the nucleotides in the central region comprise a 2'-deoxy sugar moiety.

5. The antisense oligonucleotide according to claim 4, wherein the antisense oligonucleotide has a sugar motif selected from the group consisting of the following (5' to 3'): eekddddddddddkke, ekkddddddddddkke, kkkdyddddddddkkk, kkkddydddddddkkk, kkkdddyddddddkkk, kkkddddddddddkkk, and eeeeeeddddddddddeeeee; wherein e represents a 2'-MOE sugar moiety, k represents a cEt sugar moiety, d represents a 2'-deoxy sugar moiety, and y represents a 2'-OMe sugar moiety.

6. The antisense oligonucleotide according to any one of claims 1 to 5, wherein the antisense oligonucleotide comprises at least one modified internucleoside linkage; preferably, each internucleoside linkage of the antisense oligonucleotide is a modified internucleoside linkage; preferably, the modified internucleoside linkage is selected from phosphorothioate (PS) and phosphoramidate (PN) (e.g., methanesulfonyl phosphoramidate (MsPA)), preferably PS.

7. The antisense oligonucleotide according to any one of claims 1 to 6, wherein the antisense oligonucleotide comprises an oligonucleotide consisting of 12-30, 14-22, 14-20, 14-18, 14-20, 15-17, 15-25, or 16-20 linked nucleotides, preferably, the oligonucleotide consists of 20 contiguous nucleotides; preferably, the antisense oligonucleotide comprises, or consists of, a nucleotide sequence as set forth in any one of SEQ ID NOs: 21 to 38, or a nucleotide sequence having 1 to 3 nucleotide differences therefrom.

8. The antisense oligonucleotide according to any one of claims 1 to 7, wherein the antisense oligonucleotide comprises a ligand; preferably, wherein the ligand comprises a ligand targeting hepatocytes, preferably, the ligand comprises a galactose moiety, a galactosamine moiety, or an N-acetylgalactosamine moiety, more preferably, the ligand is a trivalent or tetravalent N-acetylgalactosamine moiety, and even more preferably, the hepatocyte-targeting ligand is L96, NAG25, or NAG37; or the ligand comprises a ligand targeting non-hepatocyte cells, preferably the ligand is a lipophilic group, an integrin ligand, or a transferrin receptor 1 ligand; the lipophilic group is preferably selected from: lipids, vitamins, steroids, C₅-C₃₀ saturated or unsaturated fatty acids, C₅-C₃₀ alkyl groups, and polypeptides comprising at least one positively charged amino acid residue; the lipophilic group is more preferably selected from cholesterol, C₁₆ saturated or unsaturated fatty acid, C₁₆ alkyl group, C₂₂ saturated or unsaturated fatty acid, or C₂₂ alkyl group.

9. An RNAi agent for inhibiting expression of sodium voltage-gated channel alpha subunit 10 (SCN10A) gene in a cell, comprising a sense strand and an antisense strand forming a duplex region, wherein the antisense strand comprises at least 15 contiguous nucleotides of a nucleotide sequence selected from SEQ ID NOs: 59 to 78 and nucleotide sequences having 1 to 3 nucleotide differences therefrom.

10. The RNAi agent according to claim 9, wherein the duplex region is 17 to 23 base pairs in length, preferably 18 to 21 base pairs, more preferably 19 base pairs.

11. The RNAi agent according to claim 9 or 10, wherein each of the sense strand and the antisense strand is 17 to 23 nucleotides in length, preferably 19 to 21 nucleotides.

12. The RNAi agent according to any one of claims 9 to 11, wherein the RNAi agent comprises one or two blunt ends, preferably one blunt end.

13. The RNAi agent according to any one of claims 9 to 12, wherein the RNAi agent comprises one or two overhangs, preferably one overhang, each overhang comprising 1 to 4 unpaired nucleotides, preferably 2 unpaired nucleotides.

14. The RNAi agent according to claim 13, wherein the overhang is located at 3'-end of the sense strand, 3'-end of the antisense strand, or simultaneously at 3'-end of the sense strand and 3'-end of the antisense strand; preferably, the overhang is located at 3'-end of the antisense strand, and more preferably, the RNAi agent has one blunt end.

15. The RNAi agent according to any one of claims 9 to 14, wherein the sense strand comprises at least 15 contiguous nucleotides of any nucleotide sequence selected from SEQ ID NOs: 39 to 58 and nucleotide sequences having 1 to 3 nucleotide differences therefrom.

16. The RNAi agent according to any one of claims 9 to 14, wherein the antisense strand has no more than 23 nucleotides and comprises a nucleotide sequence selected from SEQ ID NOs: 59 to 78; and the sense strand has no more than 21 nucleotides and comprises a nucleotide sequence from SEQ ID NOs: 39 to 58.

17. The RNAi agent according to claim 16, wherein
the sense strand comprises or consists of a sequence set forth in SEQ ID NO:39 or a nucleotide sequence having 1, 2, or 3 nucleotide differences therefrom, and the antisense strand comprises or consists of a sequence set forth in SEQ ID NO:59 or a nucleotide sequence having 1, 2, or 3 nucleotide differences therefrom;
the sense strand comprises or consists of a sequence set forth in SEQ ID NO:40 or a nucleotide sequence having 1, 2, or 3 nucleotide differences therefrom, and the antisense strand comprises or consists of a sequence set forth in SEQ ID NO:60 or a nucleotide sequence having 1, 2, or 3 nucleotide differences therefrom;
the sense strand comprises or consists of a sequence set forth in SEQ ID NO:41 or a nucleotide sequence having 1, 2, or 3 nucleotide differences therefrom, and the antisense strand comprises or consists of a sequence set forth in SEQ ID NO:61 or a nucleotide sequence having 1, 2, or 3 nucleotide differences therefrom;
the sense strand comprises or consists of a sequence set forth in SEQ ID NO:42 or a nucleotide sequence having 1, 2, or 3 nucleotide differences therefrom, and the antisense strand comprises or consists of a sequence set forth in SEQ ID NO:62 or a nucleotide sequence having 1, 2, or 3 nucleotide differences therefrom;
the sense strand comprises or consists of a sequence set forth in SEQ ID NO:43 or a nucleotide sequence having 1, 2, or 3 nucleotide differences therefrom, and the antisense strand comprises or consists of a sequence set forth in SEQ ID NO:63 or a nucleotide sequence having 1, 2, or 3 nucleotide differences therefrom;
the sense strand comprises or consists of a sequence set forth in SEQ ID NO:44 or a nucleotide sequence having 1, 2, or 3 nucleotide differences therefrom, and the antisense strand comprises or consists of a sequence set forth in SEQ ID NO:64 or a nucleotide sequence having 1, 2, or 3 nucleotide differences therefrom;
the sense strand comprises or consists of a sequence set forth in SEQ ID NO:45 or a nucleotide sequence having 1, 2, or 3 nucleotide differences therefrom, and the antisense strand comprises or consists of a sequence set forth in SEQ ID NO:65 or a nucleotide sequence having 1, 2, or 3 nucleotide differences therefrom;
the sense strand comprises or consists of a sequence set forth in SEQ ID NO:46 or a nucleotide sequence having 1, 2, or 3 nucleotide differences therefrom, and the antisense strand comprises or consists of a sequence set forth in SEQ ID NO:66 or a nucleotide sequence having 1, 2, or 3 nucleotide differences therefrom;
the sense strand comprises or consists of a sequence set forth in SEQ ID NO:47 or a nucleotide sequence having 1, 2, or 3 nucleotide differences therefrom, and the antisense strand comprises or consists of a sequence set forth in SEQ ID NO:67 or a nucleotide sequence having 1, 2, or 3 nucleotide differences therefrom;
the sense strand comprises or consists of a sequence set forth in SEQ ID NO:48 or a nucleotide sequence having 1, 2, or 3 nucleotide differences therefrom, and the antisense strand comprises or consists of a sequence set forth in SEQ ID NO:68 or a nucleotide sequence having 1, 2, or 3 nucleotide differences therefrom;
the sense strand comprises or consists of a sequence set forth in SEQ ID NO:49 or a nucleotide sequence having 1, 2, or 3 nucleotide differences therefrom, and the antisense strand comprises or consists of a sequence set forth in SEQ ID NO:69 or a nucleotide sequence having 1, 2, or 3 nucleotide differences therefrom;
the sense strand comprises or consists of a sequence set forth in SEQ ID NO:50 or a nucleotide sequence having 1, 2, or 3 nucleotide differences therefrom, and the antisense strand comprises or consists of a sequence set forth in SEQ ID NO:70 or a nucleotide sequence having 1, 2, or 3 nucleotide differences therefrom;
the sense strand comprises or consists of a sequence set forth in SEQ ID NO:51 or a nucleotide sequence having 1, 2, or 3 nucleotide differences therefrom, and the antisense strand comprises or consists of a sequence set forth in SEQ ID NO:71 or a nucleotide sequence having 1, 2, or 3 nucleotide differences therefrom;
the sense strand comprises or consists of a sequence set forth in SEQ ID NO:52 or a nucleotide sequence having 1, 2, or 3 nucleotide differences therefrom, and the antisense strand comprises or consists of a sequence set forth in SEQ ID NO:72 or a nucleotide sequence having 1, 2, or 3 nucleotide differences therefrom;
the sense strand comprises or consists of a sequence set forth in SEQ ID NO:53 or a nucleotide sequence having 1, 2, or 3 nucleotide differences therefrom, and the antisense strand comprises or consists of a sequence set forth in SEQ ID NO:73 or a nucleotide sequence having 1, 2, or 3 nucleotide differences therefrom;
the sense strand comprises or consists of a sequence set forth in SEQ ID NO:54 or a nucleotide sequence having 1, 2, or 3 nucleotide differences therefrom, and the antisense strand comprises or consists of a sequence set forth in SEQ ID NO:74 or a nucleotide sequence having 1, 2, or 3 nucleotide differences therefrom;
the sense strand comprises or consists of a sequence set forth in SEQ ID NO:55 or a nucleotide sequence having 1, 2, or 3 nucleotide differences therefrom, and the antisense strand comprises or consists of a sequence set forth in SEQ ID NO:75 or a nucleotide sequence having 1, 2, or 3 nucleotide differences therefrom;
the sense strand comprises or consists of a sequence set forth in SEQ ID NO:56 or a nucleotide sequence having 1, 2, or 3 nucleotide differences therefrom, and the antisense strand comprises or consists of a sequence set forth in SEQ ID NO:76 or a nucleotide sequence having 1, 2, or 3 nucleotide differences therefrom;
the sense strand comprises or consists of a sequence set forth in SEQ ID NO:57 or a nucleotide sequence having 1, 2, or 3 nucleotide differences therefrom, and the antisense strand comprises or consists of a sequence set forth in SEQ ID NO:77 or a nucleotide sequence having 1, 2, or 3 nucleotide differences therefrom; or
the sense strand comprises or consists of a sequence set forth in SEQ ID NO:58 or a nucleotide sequence having 1, 2, or 3 nucleotide differences therefrom, and the antisense strand comprises or consists of a sequence set forth in SEQ ID NO:78 or a nucleotide sequence having 1, 2, or 3 nucleotide differences therefrom.

18. The RNAi agent according to claim 17, wherein the RNAi agent comprises duplex 51059, 51069, 51071, 51078, 51089, 51120, 51154, 51201, 51264, 51284, 51286, 51329, 51346, 51350, 51370, 51407, 51415, 51417, 51435, or 51441.

19. The RNAi agent according to any one of claims 9 to 18, wherein the sense strand and/or antisense strand of the RNAi agent comprises at least one modified nucleotide; the modified nucleotide is independently selected from the group consisting of: 2'-deoxythymidine (dT) nucleotide, 2'-O-methyl-modified nucleotide (2'-OMe), 2'-fluoro-modified nucleotide (2'-F), 2'-deoxy-modified nucleotide, locked nucleic acid (LNA), unlocked nucleic acid (UNA), bridged nucleic acid (BNA), glycol nucleic acid (GNA), threose nucleic acid (TNA), conformationally restricted nucleotide, constrained ethyl nucleotide (cEt), 2'-amino-modified nucleotide, 2'-O-allyl-modified nucleotide, 2'-C-alkyl-modified nucleotide, 2'-O-methoxyethyl-modified nucleotide (2'-MOE), abasic nucleotide, inverted abasic nucleotide, inverted nucleotide, morpholino nucleotide (MOP), phosphoramidate (PN), tetrahydropyran-modified nucleotide (THP), 1,5-anhydrohexitol-modified nucleotide (HNA), cyclohexenyl-modified nucleotide, nucleotide comprising a phosphorothioate group (PS), nucleotide comprising a methylphosphonate group, nucleotide comprising a 5'-phosphate, nucleotide comprising a 5'-phosphate mimic, cationic lipid-conjugated nucleotide, nucleotide comprising a 5'-vinylphosphonate (5'-VP), and combinations thereof; preferably selected from 2'-O-methyl-modified nucleotide, 2'-fluoro-modified nucleotide, nucleotide comprising a phosphorothioate internucleotide linkage, and combinations thereof; and/or preferably, each nucleotide of the sense strand and/or the antisense strand of the RNAi agent is modified.

20. The RNAi agent according to claim 19, wherein in 5' to 3' direction, the nucleotides at positions 2, 5, 7, and 14 of the antisense strand are 2'-fluoro-modified nucleotides, and one of the nucleotides at positions 12 and 16 of the antisense strand is a 2'-fluoro-modified nucleotide, while the nucleotides at remaining positions of the antisense strand are all 2'-methoxy-modified nucleotides.

21. The RNAi agent according to claim 19 or 20, wherein the antisense strand comprises at least one phosphorothioate internucleotide linkage; preferably, the phosphorothioate internucleotide linkage is present at one or more of the following locations: (i) between nucleotide positions 1 and 2 at 5'-end of the antisense strand; (ii) between nucleotide positions 2 and 3 at 5'-end of the antisense strand; (iii) between nucleotide positions 1 and 2 at 3'-end of the antisense strand; and (iv) between nucleotide positions 2 and 3 at 3'-end of the antisense strand.

22. The RNAi agent according to any one of claims 19 to 21, wherein in 5' to 3' direction, the nucleotides at positions 7 and 9 of the sense strand are 2'-fluoro-modified nucleotides, and one or two of the nucleotides at positions 5, 8, and 11 of the sense strand are 2'-fluoro-modified nucleotides, while the nucleotides at remaining positions of the sense strand are all 2'-methoxy-modified nucleotides.

23. The RNAi agent according to any one of claims 19 to 22, wherein the sense strand has at least one phosphorothioate internucleoside linkage; preferably, the phosphorothioate internucleoside linkage is present at one or more of the following positions: (i) between the 1st nucleotide and the 2nd nucleotide at the 5' end of the sense strand; (ii) between the 2nd nucleotide and the 3rd nucleotide at the 5' end of the sense strand; (iii) between the 1st nucleotide and the 2nd nucleotide at the 3' end of the sense strand; and (iv) between the 2nd nucleotide and the 3rd nucleotide at the 3' end of the sense strand.

24. The RNAi agent according to claim 23, wherein the modification is selected from one of STC, ESC, Advanced ESC, ESC+, AD1-3, AD5 and GalXC.

25. The RNAi agent according to any one of claims 19 to 24, wherein the 5'-terminal nucleotide of the antisense strand contains a phosphate or phosphate analog modification, preferably 5'-VP.

26. The RNAi agent according to any one of claims 19 to 25, wherein the RNAi agent comprises a ligand targeting hepatocytes, preferably, the ligand comprises a galactose moiety, a galactosamine moiety, or an N-acetylgalactosamine moiety, more preferably, the ligand is a trivalent or tetravalent N-acetylgalactosamine moiety, and even more preferably, the hepatocyte-targeting ligand is L96, NAG25, or NAG37; alternatively, the RNAi agent comprises a ligand targeting non-hepatocyte cells, preferably a lipophilic group, an integrin ligand, or a transferrin receptor 1 ligand; the lipophilic group is preferably selected from: lipids, vitamins, steroids, C₅-C₃₀ saturated or unsaturated fatty acids, C₅-C₃₀ alkyl groups, and polypeptides comprising at least one positively charged amino acid residue; the lipophilic group is more preferably selected from cholesterol, C₁₆ saturated or unsaturated fatty acid, C₁₆ alkyl group, C₂₂ saturated or unsaturated fatty acid, or C₂₂ alkyl group.

27. A pharmaceutical composition comprising an antisense oligonucleotide according to any one of claims 1 to 8, or an RNAi agent according to any one of claims 9 to 26; and a pharmaceutically acceptable carrier; preferably, the pharmaceutical composition is formulated for intravenous or subcutaneous injection.

28. Use of an antisense oligonucleotide according to any one of claims 1 to 8, an RNAi agent according to any one of claims 9 to 26, or a pharmaceutical composition of claim 27 in the manufacturing of a medicament for:
(i) reducing expression level of SCN10A in a cell;
(ii) preventing or treating a disease mediated by aberrant expression levels of SCN10A;
or
(iii) prevention or treatment of a disease or condition selected from acute and chronic pain, such as traumatic and postoperative pain, nociceptive pain (e.g., pain caused by osteoporosis and rheumatoid arthritis), neuropathic pain (central types, such as post-stroke pain, spinal cord injury, migraine, HIV-associated neuropathic pain, etc.; peripheral types, such as postherpetic neuralgia and diabetic neuropathy), visceral pain (e.g., pain arising from pancreatitis, inflammatory bowel syndrome, etc.), and mixed pain (e.g., low back pain, cancer pain, fibromyalgia, etc.).
